# EUROPEAN PATENT SPECIFICATION

(11) **EP 2 333 046 B1**
(45) Date of publication and mention of the grant of the patent: **11.03.2020**
(21) Application number: 09819040.8
(22) Date of filing: 16.07.2009
(51) Int. Cl.: C12N 1/20, C05F 17/00, C05G 5/00, A01G 31/00, C05F 3/00, C05F 11/10, C12N 1/14, A01G 22/00

(54) **METHOD FOR PRODUCTION OF SEED MATERIAL FOR MICROORGANISMS OPTIMIZED AS CATALYST FOR PARALLEL COMPLEX MINERALIZATION REACTION**
VERFAHREN ZUR HERSTELLUNG EINES ZUR VERWENDUNG ALS KATALYSATOR FÜR PARALLELE UND KOMPLEXE MINERALISIERUNGSREAKTIONEN OPTIMIERTEN SAATMATERIALS FÜR MIKROORGANISMEN
PROCÉDÉ POUR LA PRODUCTION DE MATÉRIAU D'INOCULATION POUR DES MICRO-ORGANISMES OPTIMISÉ EN TANT QUE CATALYSEUR POUR UNE RÉACTION DE MINÉRALISATION COMPLEXE PARALLÈLE

(30) Priority: 09.10.2008 JP 2008262390
(43) Date of publication of application: 15.06.2011
(73) Proprietor: National Agriculture and Food Research Organization, Tsukuba-shi, Ibaraki 305-8517 (JP)
(72) Inventor: SHINOHARA Makoto, Tsu-shi Mie 514-2392 (JP)
(74) Representative: Gille Hrabal
(86) International application number: PCT/JP2009/062870
(87) International publication number: WO 2010/041503

(56) References cited:
- GB-A- 2 181 424
- GB-A- 2 431 926
- JP-A- 2007 119 260
- US-B1- 6 572 773
- SHIN SHINOHARA: '''Yuki Hiryo de Yoeki Saibai. . . Kashika suru `Ne '''' KAGAKU TO SEIBUTSU vol. 46, no. 4, 01 April 2008, pages 230 - 232, XP008141100

## Description

### Technical Field

The present invention relates to a method of producing an inoculum of microorganisms optimized as a catalyst for a multiple parallel mineralization.

The present invention also relates to a method of producing a fertilizer containing nitrate nitrogen as inorganic nutrients using the inoculum.

### Background Art

In recent years, movements of reducing the use of a chemical fertilizer and promoting the use of an organic fertilizer have been active worldwide from the viewpoint that a recycling society should be established.

Also in 'hydroponics' without the use of soil, which is increasingly used in the production of, for example, vegetables such as a tomato and flower and ornamental plants, expectations for the use of the organic fertilizer have been increased.

However, the utilization of the organic fertilizer in hydroponics has heretofore been impossible. This is because the direct addition of an organic material to a nutrient solution generates a harmful intermediary metabolite, which damages the root of a plant. Hence, only the chemical fertilizer has heretofore been used in hydroponics.

Many researchers have considered that a technology for mineralizing an organic material in advance into a material easily absorbed by a plant is required to utilize the organic fertilizer in the hydroponics.

The technology for mineralizing an organic material includes a waste water treatment technology using microorganisms (for example, see Patent Literature 1).

However, the technology is not suitable for collecting nitrate nitrogen and does not match the purpose of use for a fertilizer containing nitrate nitrogen as inorganic nutrients because the technology involves a denitrification (a reaction for reducing nitrate nitrogen to release nitrogen as nitrogen gas).

In view of the foregoing, the "multiple parallel mineralization method" described in each of Patent Literature 2 and Non Patent Literature 2 has been invented as a technology capable of collecting nitrate nitrogen (as a nitrate ion) from an organic material to be utilized as inorganic nutrients.

The technology is a highly reproducible method of degrading organic nitrogen while suppressing the denitrification, and collecting a nitrate ion which is nitrate nitrogen as inorganic nutrients. The technology is used to simultaneously perform ammonification and nitrification in the same reaction solution by using sequential proliferation of microorganisms capable of conducting degradation of an organic material (ammonification) and generation of a nitrate ion (nitrification) in the same reaction system. The reaction can suppress the denitrification unlike the above-mentioned waste water treatment technology or the like.

The use of the technology of the multiple parallel mineralization method enables the use of organic fertilizers in hydroponics by direct addition, and further, application of the technology enables mineralization of an organic material into nitrate nitrogen to produce inorganic nutrients (for example, see Non Patent Literatures 1 and 2).

The invention disclosed in Patent Literature 2 attracts attention as a technology for realizing hydroponics using an organic fertilizer and for realizing production of inorganic nutrients such as nitrate nitrogen by using organic resources as raw materials. Therefore, the technology is greatly expected from farmers and plant factories which are interested in the technology as a novel hydroponics technology, companies which plan recycling of organic resources, and the like.

However, in the above-mentioned method disclosed in Patent Literature 2, there is no choice but to use a "naturally-occurring microorganism source" such as soil or water from lake and marsh as the microorganism source.

The microorganism source is not optimized for the multiple parallel mineralization, and hence, the conventional multiple parallel mineralization method has the following problems to be improved in practical use.

That is, the first problem is a long period of time to complete the reactions . This is because it is necessary to wait for sequential proliferation of microorganisms capable of conducting an ammonification reaction and microorganisms capable of conducting a nitrification reaction as the reactions are allowed to proceed from the ammonification reaction to the nitrification reaction because the "naturally-occurring microorganism source (such as soil or water from lake and marsh)" is not optimized for the multiple parallel mineralization.

Specifically, the time to complete the reactions is about two weeks or more, which may cause a trouble such as missing the proper planting time for seedlings in hydroponics.

Next, the second problem is impossibility of addition of an organic material in a large amount at one time. As mentioned above, the "naturally-occurring microorganism source" is not optimized for the multiple parallel mineralization, and hence, nitrifying microorganisms contained therein (microorganisms capable of conducting nitrification) have a low tolerance for organic components and are killed by exposure to a large amount of organic components. Therefore, in the case where the organic material is added in a large amount at one time in the 'culture process', nitrate nitrogen cannot be collected because the nitrification reaction is not allowed to proceed.

Specifically, the upper limit of the amount of the organic material added is about 2 g per addition per L of water (solution) of the reaction system. Therefore, to collect a high concentration of nitrate nitrogen, it is necessary to add the organic material in several batches (preferably daily), and hence, the procedure becomes cumbersome.

Then, the third problem is a large amount of the microorganism source added.

The problem is caused because the above-mentioned naturally-occurring microorganism source is not one (microorganism ecosystem) optimized for the multiple parallel mineralization, and hence, the nitrifying microorganisms have a very low tolerance for organic components and it is unavoidable that the nitrifying bacteria are damaged by exposure to the organic components, and the amount of the microorganism source added needs to be adjusted in consideration of loss of the microorganisms by the damages.

Specifically, it is necessary to add soil in an amount of about 5 g or more per L of water (solution) of the reaction system. If the amount is 5 g or less, there is a risk that nitrate nitrogen cannot be collected because the nitrifying microorganisms are killed by exposure to the organic components.

Further, addition of a large amount of the microorganism source is problematic in the field of hydroponics. In the field of hydroponics, tons of nutrient solutions are used, and hence, a few kilos of soil need to be added to the nutrient solutions. However, if the soil is added in such amount, a problem of clogging in a flow path by soil particle is often caused.

Moreover, if the soil is added in such amount, soil aggregates become large to make the system anaerobic in many cases . Therefore, denitrifying microorganisms which favor an anaerobic environment (microorganisms capable of conducting a denitrification) may proliferate to lose nitrate nitrogen as nitrogen gas. Moreover, the anaerobic microorganisms secrete a component undesirable (phytotoxic) for plants, which inhibits the growth of the plants.

Patent Literature 3 discloses a method of nitrification of fluids and in particular fluids to oxidize ammonia and nitrites to produce nitrates. A microbiological process is disclosed for the nitrification of a fluid comprising passing the fluid to be nitrified through an immobilized biomass comprising nitrifying bacteria, as a biofilm grown on porous particles such as coke. The document thus discloses a technology for removing toxic ammonia etc. from fluids using nitrifying bacteria.

Patent Literature 4 discloses a charred biological material comprising colonizing microbes including Nitrosomonas and Nitrobacter etc. in charcoal, as well as a method of treating environmental pollutant such as ammonia and nitrate contained in contaminated soil and water by using the material.

Patent Literature 5 discloses a filter useful for purification of water comprising Zeolite particles absorbing ammonia and a foamed plastics material suitable to host nitrifying bacteria, and discloses that the filter can be used as a plant growth medium. The document thus relates to a technology for ammonia removal treatment using nitrifying bacteria.

Therefore, development of a technology for significantly reducing the amount of the microorganism source added has been required.

Accordingly, it has been desired to develop a method of generating nitrate nitrogen as inorganic nutrients from an organic material by performing the multiple parallel mineralization at a level suitable for practical use in an efficient fashion, to thereby solve the above-mentioned three problems.
[Patent Literature 1] JP 2001-300583 A
[Patent Literature 2] JP 2007-119260 A
[Patent Literature 3] US 6,572,773 B1
[Patent Literature 4] GB 2 431 926 A
[Patent Literature 5] GB 2 181 424 A
[Non Patent Literature 1] Research Journal, 2008, 31 (1), p. 44-46
[Non Patent Literature 2] "Hydroponics using organic fertilizer", Agriculture and horticulture, Vol. 81, p. 753-764 (2006)

### Disclosure of the Invention

### Problem to be solved by the Invention

To solve the above-mentioned problems, an object of the present invention is to provide a method which can drastically reduce a time to complete a reaction for mineralizing an organic material into nitrate nitrogen and can add a large amount of an organic material at one time in a multiple parallel mineralization for generating nitrate nitrogen as inorganic nutrients from the organic material, resulting in efficient generation of a high concentration of nitrate nitrogen and drastic reduction of the amount of the microorganism source added.

### Means for solving the Problems

The inventor of the present invention has found that the use of an 'inoculum of microorganisms optimized as a catalyst for a multiple parallel mineralization' as a microorganism source can drastically reduce the time to complete a reaction for mineralizing an organic material into nitrate nitrogen and can add a large amount of an organic material at one time in the multiple parallel mineralization for generating nitrate nitrogen as inorganic nutrients from the organic material, resulting in efficient generation of a high concentration of nitrate nitrogen and drastic reduction of the amount of the microorganism source added.

It should be noted that known microorganism sources containing microorganisms capable of conducting ammonification and microorganisms capable of conducting nitrification include inoculums for tropical fish breeding (for filtration of breeding water) and activated sludge from sewage plants.

However, many of the inoculums for tropical fish breeding contain microorganisms capable of conducting a denitrification (denitrifying microorganisms) because the inoculums are originally intended to control an environment for the growth of fish and mainly used for removing nitrogen. Nitrate nitrogen is lost by the microorganisms, and hence, an inoculum capable of generating a high concentration of nitrate nitrogen has not been provided.

In addition, the activated sludge from sewage plants is mainly intended to promote the denitrification, and hence, the activated sludge contains many microorganisms capable of conducting a denitrification (denitrifying microorganisms).

Therefore, the inoculums are not "microorganisms optimized as a catalyst for the multiple parallel mineralization".

The present invention has been completed on the basis of those findings.

That is, a first aspect of the present invention is a method of producing an inoculum having the following characteristic (F) ;
(F) a characteristic such that the inoculum maintains its function as the inoculum of the microorganisms optimized as a catalyst for the multiple parallel mineralization when the inoculum is heated at 50 to 80°C,
wherein the multiple parallel mineralization is a reaction for successively performing degradation of an organic material into ammonium nitrogen and nitrification of ammonium nitrogen into nitrate nitrogen in the same reaction system as well as a reaction for generating nitrate nitrogen by degrading organic nitrogen contained in the organic material into ammonium nitrogen and then performing nitrification of ammonium nitrogen through nitrification in degradation of the organic material;
the method comprising:
placing water in a container that can store water therein, adding the following microorganism source (B) containing microorganisms capable of conducting a multiple parallel mineralization;
(B) at least one microorganism source selected from the group consisting of soil, compost, activated sludge, and water collected from nature;
wherein the microorganisms are selected from microorganisms capable of conducting ammonification and microorganisms capable of conducting nitrification;
maintaining an environment in water to satisfy all the following conditions (C1) to (C4), thereby culturing the microorganisms capable of conducting a multiple parallel mineralization;
(C1) a water temperature condition of 15 to 37°C:
(C2) a condition of maintaining an aerobic condition by aeration, shaking, or aeration and shaking:
(C3) a condition of adding the following organic material (D) in an amount of 0.01 to 2 g in terms of dry weight per L of the water per 1 to 14 days;
   (D) at least one organic material selected from the group consisting of fish-based soluble fertilizer, corn steep liquid, oil cake, fish flour, milk, soybean cake, yeast cake, sake cake, shochu cake, and raw garbage; and
(C4) a condition of stopping addition of the organic material when a concentration of a nitrate ion generated in the water reaches 10 to 50 mg/L;
forming a biofilm on the following solid surface (E) that contacts with the water and then collecting the biofilm;
(E) at least one solid surface selected from the group consisting of a wall surface of the container and a bottom surface of the container;
and utilizing the collected biofilm as an inoculum of the microorganisms optimized as a catalyst for the multiple parallel mineralization

A second aspect of the present invention is the method of producing an inoculum according to the first aspect, wherein one or more of the following materials (A1) to (A3) is immersed in the container;
(A1) a solid support comprising bamboo charcoal, charcoal, pearlite, sea sand, vermiculite, ceramic, zeolite, glass, rockwool, urethane, nylon, or a melamine resin;
(A2) a plate-like object comprising glass, acryl, plastic, ceramics, or pottery; and
(A3) a columnar structure comprising glass, acryl, plastic, ceramics, or pottery, and the biofilm is formed on the following solid surface (E-1);
   (E-1) at least one solid surface selected from the group consisting of a wall surface of the container, a bottom surface of the container, a surface of the solid support, a surface of the plate-like object, and a surface of the columnar structure.

A third aspect of the invention is a method of producing an inoculum having the following characteristic (F);
(F) a characteristic such that the inoculum maintains its function as the inoculum of the microorganisms optimized as a catalyst for the multiple parallel mineralization when the inoculum is heated at 50 to 80°C, the method comprising:
placing water in a container that can store water therein, adding an inoculum obtained by the method of producing an inoculum according to claim 1 as microorganism source;
maintaining an environment in water to satisfy all the following conditions (C1), (C2), (C3-2), and (C4), thereby culturing the microorganisms capable of conducting a multiple parallel mineralization;
   (C1) a water temperature condition of 15 to 37°C;
   (C2) a condition of maintaining an aerobic condition by aeration, shaking, or aeration and shaking:
   (C3-2) a condition of adding the following organic material (D) in an amount of 0.01 to 10 g in terms of dry weight per L of the water per 1 to 14 days;
      (D) at least one organic material selected from the group consisting of fish-based soluble fertilizer, corn steep liquid, oil cake, fish flour, milk, soybean cake, yeast cake, sake cake, shochu cake, and raw garbage; and
   (C4) a condition of stopping addition of the organic material when a concentration of a nitrate ion generated in the water reaches 10 to 50 mg/L;
   forming a biofilm on the following solid surface (E) that contacts with the water and then collecting the biofilm;
   (E) at least one solid surface selected from the group consisting of a wall surface of the container and a bottom surface of the container;
   and utilizing the collected biofilm as an inoculum of the microorganisms optimized as a catalyst for the multiple parallel mineralization.

A fourth aspect of the invention is a method of producing an inoculum according to claim 3, wherein one or more of the following materials (A1) to (A3) is immersed in the container;
(A1) a solid support comprising bamboo charcoal, charcoal, pearlite, sea sand, vermiculite, ceramic, zeolite, glass, rockwool, urethane, nylon, or a melamine resin;
(A2) a plate-like object comprising glass, acryl, plastic, ceramics, or pottery; and
(A3) a columnar structure comprising glass, acryl, plastic, ceramics, or pottery, and the biofilm is formed on the following solid surface (E-1);
   (E-1) at least one solid surface selected from the group consisting of a wall surface of the container, a bottom surface of the container, a surface of the solid support, a surface of the plate-like object, and a surface of the columnar structure.

A fifth aspect of the invention relates to the method of producing an inoculum according to the first aspect, wherein the biofilm is collected by discarding a supernatant of a culture solution obtained after culturing the microorganisms capable of conducting a multiple parallel mineralization and then collecting the biofilm formed on the solid surface.

A sixth aspect of the invention relates to the method of producing an inoculum according to the first aspect, comprising performing a drying treatment after collecting the biofilm. The seventh aspect of the invention relates to the method of producing an inoculum according to the second aspect, wherein the biofilm is collected by discarding a supernatant of a culture solution obtained after culturing the microorganisms capable of conducting a multiple parallel mineralization and then collecting the biofilm formed on the solid surface.

An eight aspect of the invention relates to the method of producing an inoculum according to the fourth aspect, comprising performing a drying treatment after collecting the biofilm.

A ninth aspect of the invention relates to an inoculum which is obtained by the method of producing an inoculum according to any one of aspects 1, 2, 3, 4, 5, 6, 7 or 8, having the following characteristics (F) and (G);
(F) a characteristic such that the inoculum maintains its function as the inoculum of the microorganisms optimized as a catalyst for the multiple parallel mineralization when the inoculum is heated at 50 to 80°C; and
(G) a characteristic such that a microorganism composition is optimized as a catalyst for the multiple parallel mineralization.

A tenth aspect of the invention relates to a method of producing a fertilizer containing nitrate nitrogen as inorganic nutrients, the method comprising:
placing water in a container that can store water therein and adding the inoculum according to claim 9;
allowing a multiple parallel mineralization to proceed in the water by maintaining an environment in the water to satisfy all the following conditions (C1), (C2), and (C3-3);
   (C1) a water temperature condition of 15 to 37°C;
   (C2) a condition of maintaining an aerobic condition by aeration, shaking, or aeration and shaking; and
   (C3-3) a condition of adding an organic material in an amount of 20 g or less in terms of dry weight per L of the water;
providing a reaction solution containing a nitrate ion at a concentration of 100 mg/L or more;
and utilizing the resultant reaction solution as a fertilizer containing nitrate nitrogen as inorganic nutrients.

An eleventh aspect of the invention relates to the method of producing a fertilizer according to the tenth aspect, wherein the inoculum is added in an amount of 0.01 g or more per L of the water.

A twelfth aspect of the invention relates to a method of cultivating a plant, comprising adding a fertilizer containing an organic material directly to the reaction solution to perform hydroponics in the reaction solution obtained according to claim 10.

### Effects of the Invention

According to the present invention, it is possible to provide the 'inoculum of microorganisms optimized as a catalyst for a multiple parallel mineralization'.

According to the present invention, it is possible to drastically reduce a time to complete a reaction for mineralizing an organic material into nitrate nitrogen and to add a large amount of an organic material at one time in the multiple parallel mineralization for generating nitrate nitrogen as inorganic nutrients from the organic material, resulting in efficient generation of a high concentration of nitrate nitrogen and drastic reduction of the amount of the microorganism source added.

Moreover, according to the present invention, it is possible to rapidly degrade organic resources or food wastes containing a large amount of nitrogen to convert the resources or the wastes into inorganic fertilizers containing nitrate nitrogen.

### Best Mode for carrying out the Invention

The present invention relates to a method of producing an inoculum of microorganisms optimized as a catalyst for a multiple parallel mineralization in the multiple parallel mineralization for generating nitrate nitrogen as inorganic nutrients from an organic material.

The present invention also relates to a method of producing a fertilizer containing nitrate nitrogen as inorganic nutrients using the inoculum.

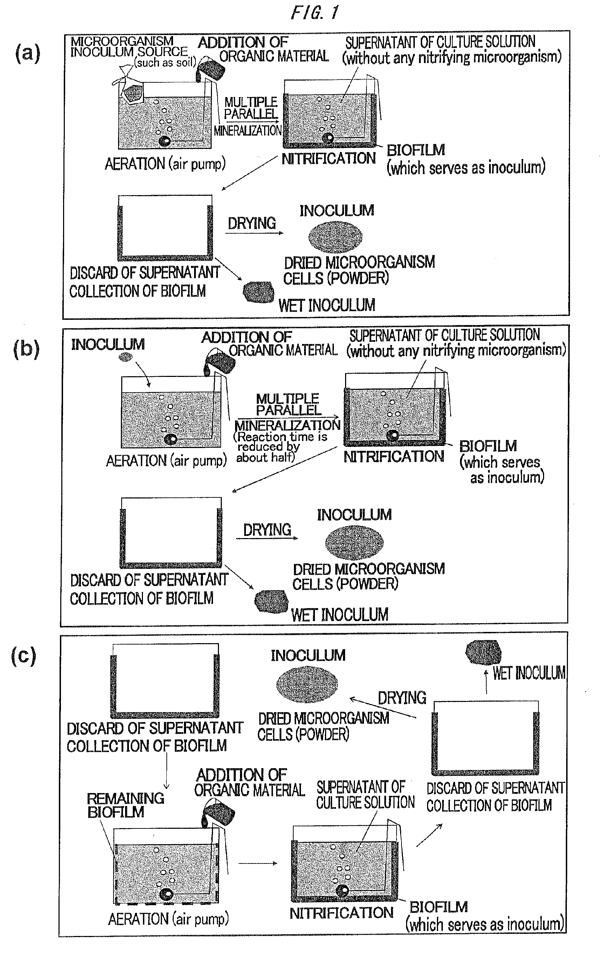

It should be noted that FIGS. 1(a) to 1(c) are diagrams illustrating embodiments of the method of producing an inoculum of microorganisms optimized as a catalyst for a multiple parallel mineralization of the present invention.

Specifically, FIG. 1(a) is a diagram illustrating an embodiment of the method using a 'naturally-occurring material (such as soil)' as the inoculum source of the microorganisms. Further, FIG. 1(b) is a diagram illustrating an embodiment of the method using 'the inoculum of the present invention' as the inoculum source of the microorganisms. Further, FIG. 1(c) is a diagram illustrating an embodiment of the method using a 'biofilm remaining on a container (solid surface)' as the inoculum source of the microorganisms.

In the present invention, the inoculum of microorganisms optimized as a catalyst for the multiple parallel mineralization, which is defined as in claim 1, is produced by: placing water in a container that can store water therein, inoculating microorganisms as defined in claim 1 capable of conducting a multiple parallel mineralization thereinto, and maintaining an environment in water to satisfy the condition (C1) to (C4) defined in claim 1 that allows the multiple parallel mineralization to proceed in the water, thereby culturing the microorganisms capable of conducting a multiple parallel mineralization; and forming a biofilm (microbial community structure) on a solid surface selected from the group consisting of a wall surface of the container and a bottom surface of the container that contacts with the water, and then utilizing the collected biofilm as an inoculum of the microorganisms optimized as catalyst for the multiple parallel mineralization.

The first step in the production of the inoculum of the microorganisms optimized as a catalyst for the multiple parallel mineralization of the present invention is a step of 'placing water in a container that can store water therein, adding microorganism source (B) defined in claim 1 containing microorganisms capable of conducting a multiple parallel mineralization for mineralizing an organic material to generate nitrate nitrogen, and maintaining an environment in water to satisfy all the conditions (C1) to (C4) defined in claim 1 thereby culturing the microorganisms capable of conducting a multiple parallel mineralization' (culture step).

It should be noted that in the culture step, the environment that allows the multiple parallel mineralization to proceed may be maintained 'after inoculating the microorganisms' or may be maintained 'after inoculating the microorganisms into water in a state in which the environment that allows the multiple parallel mineralization to proceed is maintained'.

Any container may be used as "a container that can store water therein" in this step as long as the container can store water therein.

Examples of the container include: containers which can store a relatively large amount of water therein, such as a water tank, a pot, a bucket, a tank, a water storage tank, a bathtub, and a pool; and containers which can store a relatively small amount of water therein, such as a flask, a beaker, and a test tube.

Specifically, the pot, tank, or flask may be used. Meanwhile, in the case of large-scale production or actual industrial use, the water storage tank or pool may be used.

It should be noted that, more preferably, the container is desirably one which has a structure where the solid surface area of the container that contacts with water is large with respect to the volume and where there are few parts at which water stream stagnates so as to hardly cause an anaerobic condition.

In addition, the area where the biofilm of the microorganisms is formed can be increased by placing a solid support where the microorganisms are easy to adhere in the container (by immersing the support in water in the container).

Specifically, a solid support such as bamboo charcoal, charcoal, pearlite, sea sand, vermiculite, ceramic, zeolite, glass, rockwool, urethane, nylon, or a melamine resin may be used.

Moreover, the area where the biofilm of the microorganisms is formed can be increased by immersing an immersing object such as a plate (a plate-like object) or a columnar structure in the container (by immersing the object when water is placed therein) . The immersing object desirably has a structure which is detachable and removable from water. Specifically, as the immersing object, an object made of glass, acryl, plastic, ceramics, pottery, etc., which is not putrefied and corroded in water, may be used.

As the water used in this step, tap water, distilled water, distilled pure water, well water, river water, lake water, sea water, etc. may be used.

The amount of the water is not particularly limited as long as the water is at least 50 times the dry weight of the organic material to be added. To obtain the inoculum in an adequate amount, specifically, the water is placed in the container in an amount of 0.001 to 10,000 L, preferably 0.01 to 1000 L.

In the present invention, the "multiple parallel mineralization" is a reaction for generating nitrate nitrogen by mineralizing an organic material and involves successively performing degradation of an organic material into ammonium nitrogen (ammonification) and nitrification of ammonium nitrogen into nitrate nitrogen (nitrification) in the same reaction system.

Specifically, the reaction refers to a reaction for generating nitrate nitrogen by degrading organic nitrogen contained in the organic material into ammonium nitrogen and then performing nitrification (oxidation) of ammonium nitrogen through nitrification in degradation of the organic material.

It should be noted that, in the present invention, the nitrate nitrogen generated by mineralizing the organic material includes a nitrate ion and a nitrate salt, but specifically refers to a nitrate ion.

The "microorganisms capable of conducting a multiple parallel mineralization" to be "inoculated" in this culture step include microorganisms capable of conducting ammonification and microorganisms capable of conducting nitrification, and may be ones which can conduct the multiple parallel mineralization when cultured under the below-mentioned predetermined environment.

It should be noted that the microorganisms forming the microorganisms described above are selected from microorganisms capable of conducting ammonification such as protozoans, bacteria, fungi, and any other ammonifying microorganisms; and microorganisms capable of conducting nitrification (nitrifying microorganisms) such as ammonium-oxidizing microorganisms (or nitrite-producing microorganisms) belonging to the genus *Nitrosomonas,* the genus *Nitorosococcus,* and the genus *Nitrosospira* (including the genus *Nitrosolobus* and the genus *Nitrosovibrio*) and nitrite-oxidizing microorganisms (or nitrate-producing microorganisms) belonging to the genus *Nitrobacter* and the genus *Nitrospira.*

The inoculum source of the microorganisms capable of conducting a multiple parallel mineralization in this step is specifically a "naturally-occurring material" selected from the group consisting of soil, compost including bark compost, activated sludge, or water collected from nature (specifically, water from lake and marsh, spring water, well water, river water, sea water, etc.).

However, the naturally-occurring inoculum sources are not necessarily optimized as a catalyst for a multiple parallel mineralization. Therefore, in the cases of using the above-mentioned materials as the inoculum sources, to culture the microorganisms capable of conducting a multiple parallel mineralization, specifically, it takes at least 10 days, usually 15 to 20 days to complete all the steps in production of the inoculum of the present invention in the case where the amount of an organic material added is 1 g or less per L of water and the reaction temperature is 25°C.

Therefore, the "inoculum of the microorganisms optimized as a catalyst for the multiple parallel mineralization" obtained in the present invention is preferably inoculated as the inoculum source of the microorganisms in this step.

As mentioned in detail below, the "inoculum" is a material obtained by collecting the biofilm (microbial community structure) formed on the solid surface after the culture step or a material collected so that the material contains the biofilm.

When the inoculum is added as the inoculum source of the microorganisms in this step, the culture step can be completed rapidly. Specifically, all the steps in production of the inoculum of the present invention can be completed within at most 8 days, usually 4 to 8 days in the case where the amount of an organic material added is 1 g or less per L of water and the reaction temperature is 25°C.

That is, the time period taken to complete all the steps in production of the inoculum of the present invention can be almost halved, and the production efficiency can be drastically enhanced by increasing the times of the operation.

It should be noted that the biofilm remaining on the container (solid surface) after collection of the biofilm may be used as the inoculum source in this culture step having the same functions as those of the "inoculum".

In actual production, before the "inoculum" of the present invention is obtained, the above-mentioned "naturally-occurring material " selected from the group consisting of soil, bark compost, or water collected from nature is desirably used as the inoculum source of the microorganisms in this step, and after the inoculum of the present invention is obtained, the "inoculum" is desirably used from the point of view of the efficiency as mentioned above.

In inoculation of the microorganisms in this culture step, the amount of the inoculum source added is not particularly limited, but in the case where the "naturally-occurring material" selected from the group consisting of soil, bark compost, or water collected from nature is added, a large amount of the inoculum source needs to be added to water placed in the container.

Specifically, in the case of using soil or bark compost, the inoculum source is added in an amount of 1 to 10 g with respect to 1 L of water placed in the container, and in the case of using water collected from nature, the inoculum source is added by replacing 100 to 1000 ml out of 1 L of the water placed in the container with the water collected from nature (so that the water collected from nature accounts for 10 to 100% of the total volume).

On the other hand, in the case where the "inoculum" of the present invention is added as the inoculum source, the amount of the inoculum added can be drastically reduced, and the inoculum may be added in an amount of 0.005 to 1 g in terms of dry weight per L of the water placed in the container.

Specifically, in the case of using dried microbial cells, 0.05 to 1 g of the inoculum may be added per L of the water placed in the container, and in the case of using wet microbial cells, 0.05 to 10 g of the inoculum may be added per L of the water placed in the container. Meanwhile, in the case where a mixture of the biofilm and the supernatant of a culture solution is added, the inoculum source may be added by replacing 1 to 500 ml out of 1 L of the water placed in the container with the mixture (so that the mixture accounts for 1 to 50% of the total volume).

It should be noted that, in the case where the biofilm remaining on the container (solid surface) after collection of the biofilm is used as the inoculum source, 0.005 to 1 g (in terms of dry weight) of the biofilm may be added with 1 L of water in the container to prepare water inoculated with the microorganisms.

In this culture step, the "environment that allows the multiple parallel mineralization to proceed in the water" specifically refers to an environment satisfying the conditions (C1) to (C4) defined in claim 1 where an aerobic condition is maintained in the water, an organic material is added to the water, and further the water is maintained at a water temperature of 15 to 37°C.

When such environment is maintained, the microorganisms capable of conducting a multiple parallel mineralization can be cultured.

In this culture step, when "an aerobic condition is maintained" in the water, the concentration of dissolved oxygen in the water can be increased to create a condition suitable for the activity of the microorganisms capable of conducting a multiple parallel mineralization.

Meanwhile, microorganisms capable of conducting a denitrification (denitrifying microorganisms) become active under an anaerobic condition, and hence, the above-mentioned environment is maintained for suppressing the proliferation of the microorganisms capable of conducting a denitrification.

A method of maintaining an aerobic condition in the water includes aeration, shaking, or aeration and shaking. Preferably, aeration or shaking is employed.

In this culture step, the "water temperature" suitable for allowing the multiple parallel mineralization to proceed is one suitable for growing the microorganisms capable of conducting a multiple parallel mineralization. Specifically, the water temperature is kept at 15 to 37°C, more preferably 20 to 37°C, most preferably about 25°C.

It should be noted that, in the case where the temperature is lower than 15°C, the temperature is not preferred because proliferation of the microorganisms is delayed to require a long period of time for culture. Meanwhile, in the case where the temperature is higher than 37°C, the temperature is not preferred because part of microorganisms necessary for allowing the multiple parallel mineralization to proceed may be killed.

In this culture step, the "organic material" to be added to the water may be an organic material (D) as defined in claim 1 is used. However, a nitrogen-rich organic material having a content ratio of carbon to nitrogen, a C/N ratio, of 11 or less, preferably 10 or less is desirably used because the collection efficiency of nitrate nitrogen can be enhanced.

The organic material desirably contains a protein, a protein degradation product, and an amino acid in large amounts. Specifically, at least one organic material selected from the group consisting of fish-based soluble fertilizer, corn steep liquid, oil cake, fish flour, milk, soybean cake, yeast cake, sake cake, shochu cake, and raw garbage is used as the at least one organic material. It should be noted that those are wastes obtained in a food producing process and are desirable in terms of being free of any component having toxicity.

Further, of those, it is more desirable to use fish-based soluble fertilizer, corn steep liquid, or oil cake. A specific example of the fish-based soluble fertilizer is bonito-based soluble fertilizer. Further, the corn steep liquid is, for example, corn steep liquor (CSL: corn steep liquid obtained as a by-product during producing corn starch). Further, an example of the oil cake is rapeseed oil cake.

It should be noted that the organic material may be used in a liquid form or in a powder form, but bonito-based soluble fertilizer and corn steep liquor are particularly desirable because they are liquid and easily disperse uniformly in the water.

In this culture step, a method of "adding the organic material" to the water varies depending on the type of the inoculum source of the microorganisms.

That is, in the case where a "naturally-occurringmicroorganism source" selected from the group consisting of soil, bark compost, or natural water is added as the inoculum source, to prevent killing of nitrifying microorganisms contained in the microorganism source by exposure to the organic material, it is necessary to 'gradually' add the organic material in an amount of 0.01 to 2 g (in terms of dry weight), preferably 0.05 to 1 g (in terms of dry weight) per L of the water per 1 to 14 days, preferably per 1 to 7 days, more preferably daily. For example, in the case of using rapeseed oil cake, the organic material may be added in an amount of 0.01 to 2 g.

In the case where the organic material is in a liquid form, the value of the amount in terms of dry weight may be in the range. For example, bonito-based soluble fertilizer may be added in an amount of 0.01 to 2 g in terms of liquid weight (0.007 to 1.4 g in terms of dry weight), while corn steep liquor may be added in an amount of 0.01 to 2 g in terms of liquid weight (0.005 to 1 g in terms of dry weight).

Further, in the case where the "inoculum" of the present invention is added as the inoculum source, the organic material can be added 'at one time' in an amount up to 10 g per L of water because the nitrifying microorganisms contained in the inoculum source has increased resistance to exposure to organic components.

Specifically, the inoculum is desirably added 'at the first day of culture' in an amount of 0.01 to 10 g (in terms of dry weight), preferably 0.05 to 5 g (in terms of dry weight) per L of the water. For example, rapeseed oil cake may be added in an amount of 0.01 to 10 g.

In addition, in the case where the organic material is in a liquid form, the value of the amount in terms of dry weight may be in the range. For example, bonito-based soluble fertilizer may be added in an amount of 0.01 to 10 g in terms of liquid weight (0.007 to 7 g in terms of dry weight) ; while corn steep liquor may be added in an amount of 0.01 to 10 g in terms of liquid weight (0.005 to 5 g in terms of dry weight).

In this culture step, the "time to culture" the microorganisms capable of conducting a multiple parallel mineralization is a time until the increase in the concentration of a nitrate ion in the culture solution reaches 10 to 50 mg/L.

It should be noted that, in the case where the amount of the organic material added is 1 g or less per L of water, the reaction temperature is 25°C, and the "naturally-occurring microorganism source" selected from the group consisting of soil, bark compost, or natural water is added as the inoculum source, the number of days for culture under the above-mentioned environment (culture until the increase in the concentration of a nitrate ion in the culture solution reaches 10 to 50 mg/L) is specifically 10 days at shortest, usually 15 to 20 days.

On the other hand, in the case where the amount of the organic material added is 1 g or less per L of water, the reaction temperature is 25°C, and the "inoculum" of the present invention is added, the number of days is 8 days at longest, usually 4 to 8 days.

In this culture step, in the case where the microorganisms capable of conducting a multiple parallel mineralization are cultured, the microorganisms capable of conducting a multiple parallel mineralization are cultured while suppressing proliferation of the microorganisms capable of conducting a denitrification in the biofilm formed.

The "denitrification" is a phenomenon where nitrate nitrogen is reduced into, for example, nitrogen gas or nitrous oxide gas by the microorganisms capable of conducting a denitrification (denitrifying microorganisms), thereby losing nitrate nitrogen. The reaction tends to be induced when the following two conditions are simultaneously realized: a condition where there is an organic component serving as an energy source for the denitrifying microorganisms; and a condition where nitrate nitrogen serving as an oxygen donor for the denitrifying microorganisms is generated.

Therefore, in the present invention, the microorganisms capable of conducting a multiple parallel mineralization are cultured while suppressing proliferation of the microorganisms capable of conducting a denitrification (denitrifying microorganisms) by stopping addition of the organic material before or immediately after nitrate nitrogen starts to be generated in the water.

Specifically, when (before or immediately after) the concentration of nitrate nitrogen generated in the water reaches 10 to 50 mg/L, preferably 10 to 30 mg/L in terms of a nitrate ion, the addition of the organic material is stopped.

It should be noted that the microorganisms capable of conducting a denitrification (denitrifying microorganisms) become active under an anaerobic condition, and hence, an aerobic condition is maintained in the water.

The above-mentioned culture step is performed to form the biofilm of the microorganisms capable of conducting a multiple parallel mineralization on the solid surface that contacts with the water, and then a step of collecting the biofilm (collection step) is performed.

Here, the "solid surface" that contacts with the water specifically refers to: a wall surface or a bottom surface of the container; the surface of a solid support immersed in water separately from the container; or the surface of a plate immersed in water separately from the container.

It should be noted that, from the view point of the operation efficiency of collection of the biofilm, in the case where the solid surface where the biofilm of the microorganisms is formed is the wall surface and/or bottom surface of the container, the solid surface desirably has a structure in which water stream does not often stagnate, while in the case where the solid surface is the solid support or plate immersed in water, the solid surface desirably has a structure which is detachable and removable from water.

In this step, the "collection of the biofilm" refers to collection of the biofilm formed on the solid surface or collection of a material containing the biofilm.

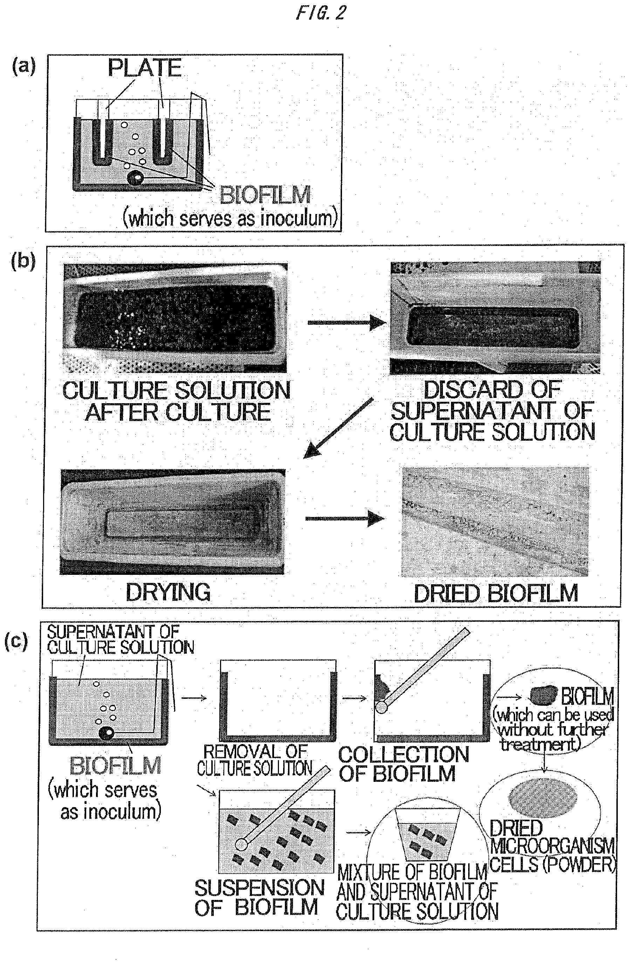

Specifically, there are the following cases: (1) the supernatant of the culture solution obtained after culturing the microorganisms capable of conducting a multiple parallel mineralization is discarded, and then the biofilm formed on the solid surface is collected; and (2) a mixture of the biofilm formed on the solid surface and the supernatant of the culture solution obtained after culturing the microorganisms capable of conducting a multiple parallel mineralization is collected. It should be noted that FIG. 2 (c) is a diagram illustrating an embodiment of the method of collecting the biofilm in the present invention.

The method (1) involves discarding the supernatant of the culture solution and collecting the biofilm formed on the solid surface.

In this case, the supernatant of the culture solution may be discarded by discharge from a drain outlet, decantation (a method of discarding the supernatant by tilting the container), aspiration, evaporative drying, or the like. The supernatant of the culture solution is desirably discarded by discharge from a drain outlet, decantation, or aspiration because a structure can be simplified and a treatment can be facilitated.

After the supernatant of the culture solution has been discarded, specifically, the biofilm may be collected and harvested by scraping the surface of the container or the surface of the solid support immersed or the immersed object. The thus-collectedbiofilm is collected as a "wet microorganism cells". Meanwhile, a support itself to which the biofilm has adhered may be collected as a wet microorganism cells of the present invention.

The method (2) involves mixing the biofilm formed on the solid surface and the supernatant of the culture solution obtained after culturing the microorganisms capable of conducting a multiple parallel mineralization and then collecting the "mixture".

In this case, specifically, mixing of the biofilm formed and the supernatant of the culture solution may be performed by: mixing the formed biofilm physically peeled off by scraping the biofilm using a brush, a wiper, or the like and the supernatant of the culture solution well; mixing the biofilm peeled off by water stream and the supernatant; or mixing the biofilm peeled off by vibrating the whole of the container and the supernatant.

In addition, in the mixture, the amount of the supernatant of the culture solution is preferably about 0.5 to 100 ml per g of the biofilm.

The "biofilm" formed in the above-mentioned culture step contains the microorganisms capable of conducting a multiple parallel mineralization at a high concentration and contains the microorganisms capable of conducting ammonification and the microorganisms capable of conducting nitrification (nitrifying microorganisms) at a high concentration. In particular, the biofilm is suitable for collecting the microorganisms capable of conducting nitrification (nitrifying microorganisms), which colonize on the solid surface.

It should be noted that the "supernatant of the culture solution" obtained after culturing the microorganisms capable of conducting a multiple parallel mineralization contains the microorganisms capable of conducting ammonification of the microorganisms capable of conducting a multiple parallel mineralization but contains few microorganisms capable of conducting nitrification (nitrifying microorganisms).

Therefore, "only the supernatant of the culture solution" has very low activity toward the nitrification reaction, and is not suitable as the inoculum of the microorganisms capable of conducting a multiple parallel mineralization.

That is, in the present invention, as in the above-mentioned method (1) or (2), the collected biofilm formed on the solid surface or the collected material containing the biofilm may be used as the "inoculum of the microorganisms optimized as a catalyst for the multiple parallel mineralization".

It should be noted that, in view of preservation of the inoculum or problems in transportation, a method involving using the biofilm collected by the method (1) is desirably employed because the volume or weight can be easily reduced.

Meanwhile, in the case where preservation or transportation is not particularly considered, the method (2) involving mixing the biofilm and the supernatant of the culture solution is desirable because the operation of the method is the easiest.

The biofilm collected by the above-mentioned method (1) or (2) may be used as the "inoculum of the microorganisms optimized as a catalyst for the multiple parallel mineralization" of the present invention, and further, the collected biofilm may be collected as the "wet microbial cells" obtained by removing excess water by centrifugation or filtration. It should be noted that, in this treatment, both the centrifugation and filtration may be performed in combination.

In this treatment, centrifugation may be performed at such a centrifugation rate that gives microorganisms no stress (2000 to 20,000×g). Meanwhile, filtration may be performed by filtering the wet biofilm or the mixture of the biofilm and the supernatant of the culture solution using a filter paper, cloth, or the like.

It should be noted that, in this treatment, the biofilm is desirably collected as wet microbial cells with a water content of 90% or less.

In this collection step, a drying treatment may be performed to collect the inoculum of the microorganisms optimized as a catalyst for the multiple parallel mineralization in the form of 'dried microbial cells'.

In the case where this collection step is performed by the above-mentioned method (1), the drying treatment may be performed after discarding the supernatant of the culture solution. Specifically, after discarding the supernatant of the culture solution, the formed biofilm which is adhered to the solid surface may be subj ected to the drying treatment. Alternatively, the drying treatment maybe performed after collection of the wet biofilm formed.

Moreover, in the case where this collection step is performed by the above-mentioned method (1) or (2) and then excess water is removed by centrifugation or filtration, the wet microbial cells may be collected and subjected to the drying treatment, to thereby obtain dried microbial cells.

The drying treatment may be performed by air drying, dry-heat treatment, reduced-pressure drying, etc. Specifically, the drying treatment may be performed by drying the wet microbial cells with air for about 1 hour to overnight (about 6 to 14 hours), preferably about overnight (about 6 to 14 hours) under a room temperature condition (15 to 37°C).

It should be noted that, in this drying treatment, it is preferred to obtain dried microbial cells with a water content of 20% or less.

The "inoculum of the microorganisms optimized as a catalyst for the multiple parallel mineralization" of the present invention can be produced by the above-mentioned steps.

The inoculum may be produced in the form of the wet microbial cells, dried microbial cells (microorganism cells after the drying treatment), a liquid (mixture of the biofilm and the supernatant of the culture solution), a solid support to which the biofilm is attached, etc.

From the viewpoint of the preservation or distribution (to transport or preserve the inoculum in another place), heat resistance of the dry inoculum, or decrease in the inoculum dose and improvement of the operation efficiency, the inoculum is preferably produced in the form of the "dried microbial cells".

Meanwhile, in the case where the multiple parallel mineralization is allowed to proceed again at the same place, the inoculum is preferably produced in the form of the "wet microbial cells" or "liquid" because the step of producing the inoculum is simple.

The inoculumof the present inventionhas a microorganism composition including the microorganisms capable of conducting ammonification and the microorganisms capable of conducting nitrification (nitrifying microorganisms), and the amount of the microorganisms capable of conducting nitrification (nitrifying microorganisms) is ten thousand to one hundred million cells per g of the obtained inoculum.

The case where the microorganisms capable of conducting ammonification do not cooperate with the microorganisms capable of conducting nitrification without the process of the multiple parallel mineralization in the inoculum of the present invention is not preferred because the microorganisms capable of conducting nitrification are easily killed by exposure to a large amount of organic components to lose the nitrification ability, resulting in failing to allow the multiple parallel mineralization to proceed.

That is, in the inoculum of the present invention, the nitrification ability of the microorganisms capable of conducting nitrification is maintained even in the presence of the organic components because the microorganisms capable of conducting ammonification and the microorganisms capable of conducting nitrification cooperate and interact with each other.

The inoculum of the present invention is one which maintains the function as the inoculum of the microorganisms optimized as a catalyst for the multiple parallel mineralization even after heating at 50 to 80°C, preferably 50 to 60°C, more preferably 50°C. Meanwhile, the time to resist the heating is about 0.1 to 12 hours, preferably about 30 minutes.

The heat resistance is effective to avoid deactivation by an assumed high temperature in a truck or a warehouse during the transportation or preservation of the inoculum.

The inoculum of the present invention can be used as the 'microorganism source' of the "microorganisms optimized as a catalyst for the multiple parallel mineralization" in generation of nitrate nitrogen as inorganic nutrients from the organic material using the microorganisms capable of conducting a multiple parallel mineralization.

In the present invention, specifically, a "fertilizer containing nitrate nitrogen as inorganic nutrients can be produced" by 'generating nitrate nitrogen as inorganic nutrients from the organic material using the microorganisms capable of conducting a multiple parallel mineralization'.

In the present invention, production of a fertilizer containing nitrate nitrogen as inorganic nutrients (fertilizer production step) is performed by: placing water in a container that can store water therein and adding thereto 'the inoculum' of the present invention; allowing the multiple parallel mineralization to proceed in the water by maintaining such an "environment that an organic material is added in an amount of 20 g or less in terms of dry weight per L of water, the water is maintained at a water temperature of 15 to 37°C, and is maintained aerobically" by aeration, shaking, or aeration and shaking, which allows the multiple parallel mineralization to proceed in the water; providing a reaction solution containing 100 mg/L or more of a nitrate ion; and utilizing the resultant reaction solution as a fertilizer containing nitrate nitrogen as inorganic nutrients.

It should be noted that, in this fertilizer production step, the environment that allows the multiple parallel mineralization to proceed may be maintained 'after adding the inoculum' or 'after adding the inoculum to water in a state in which the environment that allows the multiple parallel mineralization to proceed is maintained' .

Any container may be used as "a container that can store water therein" in this step as long as the container can store water therein and has such a structure that dissolved oxygen is easy to spread evenly.

Examples of the container include: containers which can store a relatively large amount of water therein, such as a water tank, a pot, a bucket, a tank, a water storage tank, a bathtub, and a pool; and containers which can store a relatively small amount of water therein, such as a flask, a beaker, and a test tube.

Specifically, the pot, tank, or flask may be used. Meanwhile, in the case of large-scale production or actual industrial use, the water storage tank or pool may be used.

As the water used in this step, tap water, distilled water, distilled pure water, well water, river water, lake water, sea water, etc. may be used.

The amount of the water is not particularly limited as long as the water is at least 50 times the dry weight of the organic material to be added. To obtain a fertilizer in an adequate amount, specifically, the water is placed therein in an amount of 0.001 to 10,000 L, preferably 0.01 to 1000 L.

In this fertilizer production step, the amount of the inoculum of the present invention to be added as the ' microorganism source' is 0.01 g or more, preferably 0.2 g or more per L of the water placed in the container. In the case where the inoculum is added in an amount of only less than 0.2 g, the time to complete the multiple parallel mineralization may be delayed, which is not preferred.

It should be noted that, in a conventional method, i.e., in the case where bark compost or the like is added as the microorganism source, it is necessary to add the microorganism source in an amount of about 5 g or more per L of water.

That is, the amount of the microorganism source to be added can be drastically reduced to about one-fiftieth, preferably about one-twenty-fifth of the amount of the conventional method by using the inoculum of the present invention as the microorganism source.

In this fertilizer production step, to maintain the environment that allows the multiple parallel mineralization to proceed, it is necessary 'to maintain the environment where the organic material is added, the water is maintained at a water temperature of 15 to 37°C, and is maintained aerobically'.

If such environment is maintained, it is possible to proliferate the microorganisms optimized as a catalyst for the multiple parallel mineralization in the water to allow the multiple parallel mineralization to proceed rapidly, and further to generate nitrate nitrogen as inorganic nutrients "without being accompanied by the denitrification".

In this fertilizer production step, when "an aerobic condition is maintained" in the water, the concentration of dissolved oxygen in the water can be increased to create a condition suitable for the activity of the microorganisms capable of conducting a multiple parallel mineralization.

Meanwhile, the microorganisms capable of conducting a denitrification (denitrifying microorganisms) become active under an anaerobic condition, and hence, the above-mentioned environment is suitable for suppressing proliferation of the microorganisms capable of conducting a denitrification.

A method of maintaining an aerobic condition in the water includes aeration, shaking, and aeration and shaking. Preferably, aeration or shaking is employed.

In this fertilizer production step, the "water temperature" suitable for allowing the multiple parallel mineralization to proceed is one suitable for growing the microorganisms capable of conducting a multiple parallel mineralization. Specifically, the water temperature is kept at 15 to 37°C, preferably 20 to 37°C, more preferably about 25°C.

It should be noted that, in the case where the temperature is lower than 15°C, the temperature is not preferred because proliferation of the microorganisms is delayed to require a long period of time for the reaction. Meanwhile, in the case where the temperature is higher than 37°C, the temperature is not preferred because part of the microorganisms necessary for allowing the multiple parallel mineralization to proceed may be killed.

The "addition of the organic material" to the water in this fertilizer production step may be performed by adding the organic material in a large amount at one time because the inoculum has been optimized as a catalyst for the multiple parallel mineralization.

Specifically, the organic material is added at one time in an amount of 20 g or less, preferably 10 g or less (in terms of dry weight) per L of the water.

It should be noted that, in this step, the organic material may be added before the start of the reaction or may be added again after the start of the reaction.

It should be noted that the organic material may be added in the form of a liquid or in the form of powder.

Specifically, in the case where the organic material is in the form of a liquid, bonito-based soluble fertilizer may be added in an amount of 0.1 to 10 g (liquid weight: (0.07 to 7 g in terms of dry weight)), corn steep liquor may be added in an amount of 0.1 to 10 g (liquid weight: (0.05 to 5 g in terms of dry weight)), or rapeseed oil cake may be added in an amount of 0.1 to 10 g.

It should be noted that, in a conventional method, i.e., in the case where bark compost or the like is added as the microorganism source, the microorganism source can be added in an amount of only about 2 g or less per L of water at one time.

In the case where a material other than the inoculum is added as the 'microorganism source', when the organic material is added in an amount of more than 2 g per L of water, the nitrifying microorganisms contained therein are killed by exposure to a large amount of the organic component to lose the nitrification ability, resulting in failing to allow the multiple parallel mineralization to proceed. Therefore, the microorganism sources other than the inoculum of the present invention is not the microorganisms suitable for multiple parallel mineralization.

That is, when the inoculum of the present invention is used as the microorganism source, the amount of the organic material which may be added at one time can be increased to about ten times, preferably about five times that of the conventional method.

In this fertilizer production step, a culture solution containing nitrate nitrogen at a concentration of 100 mg/L or more, preferably 200 mg/L or more in terms of a nitrate ion is obtained within 8 days, preferably 4 to 8 days from the beginning of culture.

Moreover, to obtain a culture solution with a nitrate ion concentration of 400 mg/L, the time from the start of the reaction 'to completion of mineralization of the organic material into nitrate nitrogen' is 10 days or less, preferably 8 days or less, more preferably 4 to 8 days.

It should be noted that "to completion of mineralization of the organic material into nitrate nitrogen" refers to a time taken "until the concentration of nitrate nitrogen generated reaches a peak".

It should be noted that, in a conventional method, i.e., in the case where bark compost or the like is added as the 'microorganism source', the time from the start of the reaction "to completion of mineralization of the organic material into nitrate nitrogen" is 10 days at shortest, usually 15 days or more.

That is, when the inoculum of the present invention is used as the 'microorganism source', degradation can be performed at a rate about twice or more of that of the conventional method, and hence, it is possible 'to drastically reduce the reaction time' to completion of mineralization of the organic material into nitrate nitrogen.

In this fertilizer production step, a reaction solution containing nitrate nitrogen at a "high concentration" of 100 mg/L or more, preferably 200 mg/L or more, more preferably 400 mg/L or more in terms of a nitrate ion is obtained 'efficiently'. The reaction solution maybe collected to produce a fertilizer containing nitrate nitrogen as inorganic nutrients.

It should be noted that as the fertilizer produced, the reaction solution obtained in the above-mentioned step may be used as a stock solution without further treatment, or the reaction solution may be diluted twice to ten times or be mixed with a chemical fertilizer to produce a liquid fertilizer. Moreover, the reaction solution may be processed into a concentrate, a solid powder, or a solid tablet by drying.

The fertilizer containing nitrate nitrogen as inorganic nutrients, obtained in this fertilizer production step, may be used as a fertilizer for cultivating any plants including vegetables, fruits, flower and ornamental plants, foliage plants, etc.

In particular, the fertilizer can be suitably used for cultivating: leaf vegetables such as Chinese cabbage, komatsuna, lettuce, or spinach; fruit vegetables from which fruits are harvested such as tomato; fruit trees; trees; or flower and ornamental plants. More particularly, the fertilizer can be suitably used for cultivating Chinese cabbage or komatsuna.

It should be noted that the fertilizer containing nitrate nitrogen as inorganic nutrients may be used for general plant cultivation such as hydroponics or cultivation using soil.

Further, in the present invention, it is possible 'to perform hydroponics by directly adding a fertilizer containing an organic material', which has been difficult to be achieved by a conventional method, by supplying 'the reaction solution' obtained in the fertilizer production step to a nutrient solution for the hydroponics.

Specifically, direct addition of the fertilizer containing the organic material to the nutrient solution can be performed by directly performing the above-mentioned step, i.e., production of a fertilizer, in the nutrient solution for the hydroponics.

Meanwhile, if the container (reaction tank) for the fertilizer production step is used as an apparatus for the hydroponics, the hydroponics can be performed by directly adding the fertilizer containing the organic material.

It should be noted that, in the hydroponics method, in the case where conventional bark compost or the like is used as the 'microorganism source' to construct a microorganism ecosystem in the nutrient solution, although it is necessary to add the microorganism source in an amount of about 5 g or more per L, the organic material can be added in an amount of only about 2 g per L at one time, and it takes usually 15 to 20 days or more to complete the reaction (because it takes a time to establish the microorganism ecosystem in the nutrient solution).

On the other hand, when the inoculum of the present invention is added as the 'microorganism source', it is possible to reduce the amount of 'microorganism source' added to about one-fiftieth, preferably about one-twenty-fifth of the amount of the conventional method, to add at most 10 g of the organic material at one time, and to almost halve the number of days to complete the reaction (8 days or less, preferably 4 to 8 days).

The hydroponics method may be used for cultivating any plants including vegetables, fruits, flower and ornamental plants, foliage plants, etc.

In particular, the method can be suitably used for cultivating: leaf vegetables such as Chinese cabbage, komatsuna, lettuce, or spinach; fruit vegetables from which fruits are harvested such as tomato; fruit trees; trees; or flower and ornamental plants. More particularly, the method can be suitably used for cultivating Chinese cabbage or komatsuna.

### Examples

Hereinafter, the present invention is described in more detail by way of examples.

### Example 1 (Production of inoculum: formation and collection of biofilm)

As the step of producing the inoculum of the microorganisms optimized as a catalyst for the multiple parallel mineralization, the microorganisms capable of conducting a multiple parallel mineralization were 'cultured' to form a biofilm, and the biofilm was 'collected' (culture step and collection step).

10 L of water were placed in a Wagner pot (manufactured by Fujiwara Scientific Company Co., Ltd.), and bark compost (product name Golden Bark, manufactured by Shimizu Port Lumber Industry Co-operative Association) was added thereto in an amount of 5 g per L of water.

Bonito-based soluble fertilizer (by-product from a dried bonito factory) was added in an amount of about 1 g per L of water (gradually added) daily, and the microorganisms capable of conducting a multiple parallel mineralization were cultured for two weeks at a water temperature of 25°C while an aerobic condition was maintained in the mixture by aeration using an air pump. After culture, formation of a biofilm was observed on the wall of the container. Then, the supernatant of the culture solution obtained after culture was removed by decantation and discarded.

Next, the biofilm formed on the wall of the container was air-dried overnight and collected by scraping the biofilm using a metal spatula, to thereby obtain dried microbial cells (Product 1-1 of the present invention).

FIG. 1(a) is a schematic diagram illustrating the method of forming and collecting the biofilm in this example. Moreover, FIGS. 2 are photographic images showing the process of forming and collecting the biofilm in this example.

Further, after the supernatant of the culture solution was removed and discarded by decantation in the above-mentioned step, the biofilm formed on the wall of the container was scraped using a brush without air-drying and mixed with the supernatant of the culture solution, and the mixture was collected and centrifuged to remove excess water, to thereby obtain wet microbial cells as precipitates (Product 1-2 of the present invention).

### Example 2 (Amount of inoculum added and reaction time)

The biofilm formed in the process of the multiple parallel mineralization was examined whether or not the biofilm can be used as a novel microorganism source for the multiple parallel mineralization.

50 mL of distilled pure water were placed in a flask (200-ml volume), and the dried microbial cells obtained in Example 1 (Product 1-1 of the present invention) were added as the microorganism source in an amount of 0.2 g, 0.4 g, or 1.0 g per L of water.

Bonito-based soluble fertilizer (by-product from a dried bonito factory) was added in an amount of 1 g per L of water, and the mixture was allowed to react for 16 days at a water temperature of 25°C while an aerobic condition was maintained in the mixture by shaking at 120 rpm.

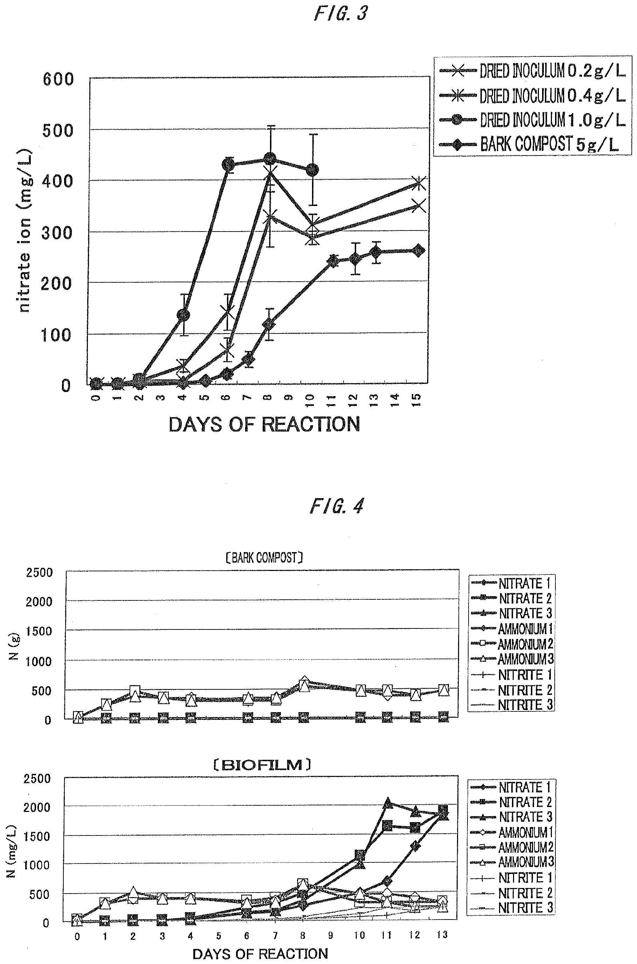

It should be noted that a control experiment was simultaneously performed by adding bark compost (product name Golden Bark, manufactured by Shimizu Port Lumber Industry Co-operative Association) as the microorganism source in an amount of 5 g per L of water to perform a reaction. FIG. 3 illustrates the results.

The results reveal that, in the case where the dried microbial cells obtained in Example 1 (Product 1-1 of the present invention) were added as the microorganism source, the reaction time to complete mineralization of the organic material into nitrate nitrogen (time until the concentration of nitrate ion reached the peak) was 6 to 8 days.

On the other hand, in the case of the control experiment, where the bark compost was added as the microorganism source, the reaction time was found to be 13 days.

Therefore, when the dried microbial cells obtained in Example 1 are added as the microorganism source, the reaction time to complete mineralization of the organic material into nitrate nitrogen can be reduced to thereby almost halve the number of days required in the case of addition of the bark compost as the microorganism source.

It should be noted that, in the case of the conventional method, i. e. , in the case of using a microorganism source not optimized for the multiple parallel mineralization such as soil or bark compost, it is necessary to add a large amount of the microorganism source because nitrifying microorganisms contained in the microorganism source are sensitive to exposure to the organic component and may be killed under a condition where a relatively large amount of the organic material is added (if a small amount of the microorganism source is added, the nitrifying microorganisms are killed to inhibit nitrification). Specifically, in the case where the organic material is added in an amount of 1 g per L of water, it is necessary to add the microorganism source in an amount of about 5 g per L of water. However, if the microorganism source is added excessively (specifically, in the case where soil or the like is added in an amount of more than 10 g per L of water), the microorganisms source itself becomes aggregates to make the mixture anaerobic, which promotes proliferation of the denitrifying microorganisms.

However, this example shows that, in the case where the biofilm formed in the process of the multiple parallel mineralization is added as the microorganism source, the multiple parallel mineralization can be performed without any difficulty even if the amount of the biofilm added is 0.2 g per L of water and that the time to complete the reaction can be reduced compared with the case where a conventional microorganism source is added (in the case of using Product 1-1 of the present invention, the time was reduced from 14 days to 8 days).

That is, it was found that it is possible to reduce the amount of the microorganism source added to about 4% of that of the conventional method and to reduce the reaction time almost by half.

The results reveal that the dried microbial cells as Product 1-1 of the present invention can be used as the "inoculum of the microorganisms optimized as a catalyst for the multiple parallel mineralization".

### Example 3 (Addition of large amount of organic material)

An experiment was performed to examine whether or not "a large amount of the organic material can be added at one time" in the case of using the biofilm formed in the process of the multiple parallel mineralization as the microorganism source.

50 mL of distilled pure water were placed in a flask (200-ml volume), and the wet microbial cells obtained in Example 1 (Product 1-2 of the present invention) were added as the microorganism source in an amount of 5 g per L of water.

Bonito-based soluble fertilizer (by-product from a dried bonito factory) was added in an amount of '10 g per L of water', and the mixture was allowed to react for 14 days at a water temperature of 25°C while an aerobic condition was maintained in the mixture by shaking at 120 rpm.

It should be noted that a control experiment was simultaneously performed by adding bark compost (product name Golden Bark, manufactured by Shimizu Port Lumber Industry Co-operative Association) as the microorganism source in an amount of 5 g per L of water to perform a reaction. FIGS. 4 illustrate the results.

The results reveal that, in the case where the wet microbial cells obtained in Example 1 (Product 1-2 of the present invention) were added as the microorganism source, the microorganisms optimized as a catalyst for the multiple parallel mineralization were proliferated without any difficulty to generate nitrate nitrogen as inorganic nutrients from the organic material even if bonito-based soluble fertilizer (by-product from a dried bonito factory) was added in an amount of '10 g per L of water' (addition of the organic material in a large amount).

On the other hand, in the case of a control experiment where bark compost was added as the microorganism source, generation of ammonium was confirmed, but generation of a nitrate ion (nitrate nitrogen) was not detected. This shows that, in the control experiment, the reaction was stopped at the time of completion of ammonification, and a nitrification reaction was not performed (the multiple parallel mineralization did not proceed until a final product was obtained).

It should be noted that, in the case of the conventional method, i.e., in the case of using soil or bark compost, which is not optimized for the multiple parallel mineralization, as the microorganism source, the amount of the organic material which can be added is up to 'about 2 g per L of water' even if the amount of the microorganism source added is increased.

However, this example shows that, in the case where the biofilm formed in the process of the multiple parallel mineralization is added as the microorganism source, the multiple parallel mineralization is performed without any difficulty to generate nitrate nitrogen as inorganic nutrients from the organic material even if a large amount of the organic material (the amount is about five times that in the case of the conventional method) is added.

### Example 4 (Multiple parallel mineralization by wet microbial cells and reaction rate thereof)

An experiment was performed to examine the rate of the multiple parallel mineralization in the case of using 'wet microbial cells' of the biofilm formed in the process of the multiple parallel mineralization as the microorganism source.

50 mL of distilled pure water were placed in a flask (200-ml volume), and the wet microbial cells obtained in Example 1 (Product 1-2 of the present invention) were added as the microorganism source in an amount of 5 g per L of water.

Bonito-based soluble fertilizer (by-product from a dried bonito factory) was added in an amount of 1 g per L of water, and the mixture was allowed to react for 16 days at a water temperature of 25°C while an aerobic condition was maintained in the mixture by shaking at 120 rpm.

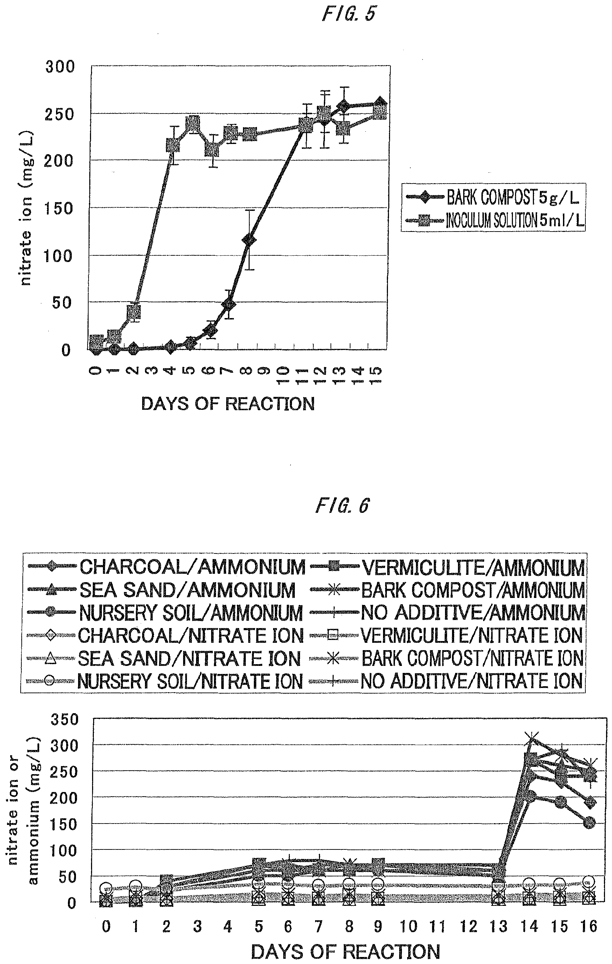

It should be noted that a control experiment was simultaneously performed by adding bark compost (product name Golden Bark, manufactured by Shimizu Port Lumber Industry Co-operative Association) as the microorganism source in an amount of 5 g per L of water to perform a reaction. FIG. 5 illustrates the results.

The results reveal that, in the case where the wet microbial cells obtained in Example 1 (Product 1-2 of the present invention) were added as the microorganism source, the reaction time to complete mineralization of the organic material into nitrate nitrogen (time until the concentration of nitrate ion reached the peak) was 4 days.

On the other hand, in the case of the control experiment, where the bark compost was added as the microorganism source, the reaction time was found to be 11 days.

Therefore, the results of this example reveal that, in the case where the biofilm optimized for the multiple parallel mineralization as the microorganism source is added in the form of the "wet microbial cells", the reaction time to complete mineralization of the organic material into nitrate nitrogen can be reduced to about one third the number of days required in the case of using a microorganism source not optimized for the multiple parallel mineralization, such as bark compost.

Comparative Example 1 (Supernatant of culture solution is not suitable as inoculum)

An examination was performed to examine whether or not the multiple parallel mineralization can be performed by using the 'supernatant of the culture solution' obtained after culturing the microorganisms capable of conducting a multiple parallel mineralization as the microorganism source.

First, the microorganisms capable of conducting a multiple parallel mineralization were cultured in the same way as in Example 1 except that CSL (product name "Yuki-no-Ekihi", manufactured by Sakata Seed Corporation) was added as the organic material in an amount of 1 g per L of water. Then, the supernatant of the culture solution obtained after culture was collected gently using a pipette (Comparative product 1).

Next, six flasks having added thereto 0.5 g of solid supports, i.e., bamboo charcoal, pearlite, sea sand, bark compost, and nursery soil (Nae-ichiban) and no solid support, respectively, were prepared and 50 ml of distilled water were added, followed by sterilization in an autoclave.

Then, 0.5 ml (10 ml per L of water) of the above-mentioned supernatant after culture (Comparative product 1) was added as the microorganism source to the respective flasks.

CSL (product name "Yuki-no-Ekihi", manufactured by Sakata Seed Corporation) was added in an amount of 0.5 g (10 g per L of water), and the mixture was allowed to react for 17 days at a water temperature of 25°C while an aerobic condition was maintained in the mixture by shaking at 120 rpm. FIG. 6 illustrates the results.

As a result, although the solid supports, on which the microorganisms capable of conducting nitrification (nitrifying microorganisms) were considered to be adhered easily, were added, only ammonification was conducted in all the flasks, and generation of a nitrate ion (nitrate nitrogen) was not confirmed.

This suggests that few microorganisms capable of conducting nitrification (nitrifying microorganisms) were suspended in the supernatant of the culture solution obtained after culturing the microorganisms capable of conducting a multiple parallel mineralization, and the microorganisms were considered to be killed by exposure to the organic component added because, even if the supernatant contained the microorganisms, the amount of the microorganisms was very small.

Therefore, the supernatant of the culture solution obtained after culturing the microorganisms capable of conducting a multiple parallel mineralization was found to be unsuitable as the inoculum of the microorganisms capable of catalyzing the multiple parallel mineralization.

### Example 5 (Method of using mixture of supernatant of culture solution after culture and biofilm as inoculum, and type of organic material in production of inoculum)

A 'mixture' obtained by mixing the supernatant of the culture solution obtained after culturing the microorganisms capable of conducting a multiple parallel mineralization and the biofilm formed was used as the microorganism source to perform the multiple parallel mineralization. Meanwhile, at the same time, an experiment was performed to examine whether or not, in the case where the multiple parallel mineralization was allowed to proceed by adding an 'organic material other than bonito-based soluble fertilizer' , the inoculum of the microorganisms optimized as a catalyst for the multiple parallel mineralization was able to be produced in the same way as in the case of using bonito-based soluble fertilizer.

First, the microorganisms capable of conducting a multiple parallel mineralization were cultured in the same way as in Example 1 except that rapeseed oil cake (manufactured by Rinoru Oil Mills Co., Ltd.) was added in an amount of about 1 g per L of water as the organic material. Then, the biofilm formed on the wall was physically peeled off by scraping using a brush and mixed well with the supernatant of the culture solution, and the mixture (Product 5 of the present invention) was collected.

Next, 9 L of water were placed in a Wagner pot (manufactured by Fujiwara Scientific Company Co., Ltd.), and 1 L of the mixture (Product 5 of the present invention) obtained in the above-mentioned steps (culture step and collection step) was added thereto as the microorganism source.

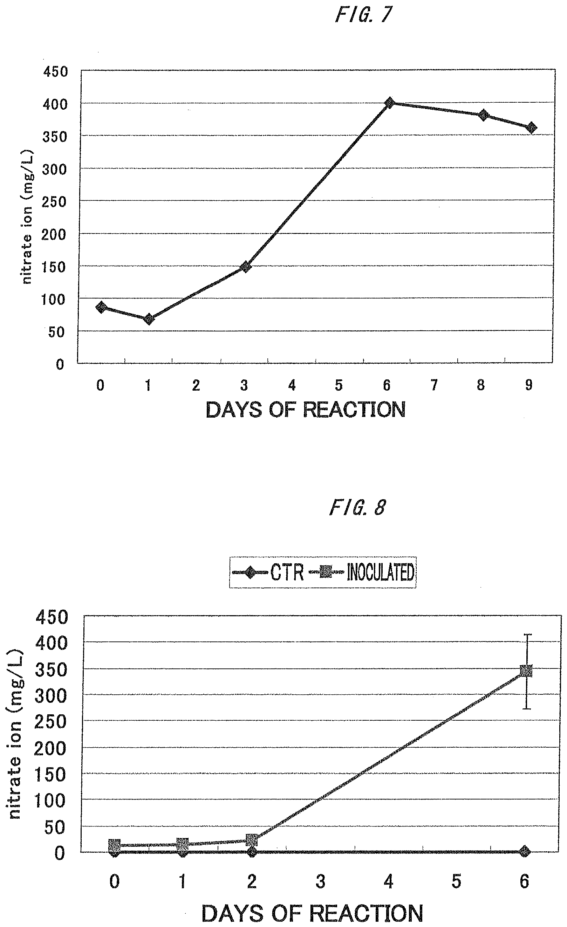

Powder of rapeseed oil cake (manufactured by Rinoru Oil Mills Co., Ltd.) was added in an amount of 10 g (1 g per L of water), and the mixture was allowed to react for 10 days at a water temperature of 25°C while an aerobic condition was maintained in the mixture by aeration using an air pump. FIG. 7 illustrates the results.

The results reveal that, in the case where the above-mentioned mixture (Product 5 of the present invention) was added as the microorganism source, the reaction time to complete mineralization of the organic material into nitrate nitrogen (time until the concentration of nitrate ion reached the peak) was 6 days. Meanwhile, a nitrate ion was generated at a concentration of more than 350 mg/L in the reaction solution after completion of the reaction.

This suggests that the mixture of the supernatant of the culture solution obtained after culture and the biofilm formed can be used as the "inoculum optimized as the microorganism source capable of catalyzing the multiple parallel mineralization".

Moreover, the results show that a biofilm obtained by using a material other than bonito-based soluble fertilizer can also be used as the inoculum.

### Example 6 (Filtration of mixture of supernatant of culture solution after culture and biofilm)

Wet microbial cells obtained by filtering the mixture of the biofilm formed after culturing the microorganisms capable of conducting a multiple parallel mineralization and the supernatant of the culture solution obtained were used as the microorganism source to perform the multiple parallel mineralization.

First, the microorganisms capable of conducting a multiple parallel mineralization were cultured in the same way as in Example 1. Then, the biofilm formed on the wall was physically peeled off by scraping using a brush and mixed well with the supernatant of the culture solution, and 400 ml of the mixture were filtered using filter paper (manufactured by Toyo Roshi Kaisha, Ltd.), followed by air-drying, to thereby obtain 0.15 g of dried microbial cells (Product 7 of the present invention).

Next, the dried microbial cells (Product 7 of the present invention) were suspended in 200 ml of distilled pure water, and 50 mL of the suspension (containing wet microorganism cells in an amount of 37.5 mg per L of water) were placed in a flask.

Bonito-based soluble fertilizer (manufactured by Makurazaki Fisheries Cooperative Associations) was added in an amount of 0.05 g (1 g per L of water), and the mixture was allowed to react for 6 days at a water temperature of 25°C while an aerobic condition was maintained in the mixture by shaking at 120 rpm. FIG. 8 illustrates the results.

As a result, in the case where the dried microbial cells (Product 7 of the present invention) obtained by filtering the mixture of the supernatant of the culture solution and the biofilm in the above-mentioned steps were added as the microorganism source, generation of a nitrate ion (nitrate nitrogen) was observed 6 days after the start of the reaction.

This suggests that the dried microbial cells obtained by filtering the mixture of the supernatant of the culture solution and the biofilm can be used as the 'inoculum of the microorganisms optimized as a catalyst for the multiple parallel mineralization' .

### Example 7 (Heat resistance of inoculum)

An experiment was performed to examine the degree of the effect of a 'heat treatment' on the activity of the inoculum of the microorganisms optimized as a catalyst for the multiple parallel mineralization.

100 mg of each of the dried microbial cells obtained in Example 1 (Product 1-1 of the present invention) were left at rest at the respective temperatures including normal temperature (about 25°C), 50°C, and 80°C for 30 minutes.

Next, 30mLof distilled pure water were placed in flasks (200-ml volume), and 30 mg (1 g per L of water) each of the dried microbial cells after the above-mentioned heat treatment were added thereto as the microorganism source.

Bonito-based soluble fertilizer (manufactured by Makurazaki Fisheries Cooperative Associations) was added in an amount of 0.03 g (1 g per L of water), and the mixture was allowed to react for 15 days at a water temperature of 25°C while an aerobic condition was maintained in the mixture by shaking at 120 rpm.

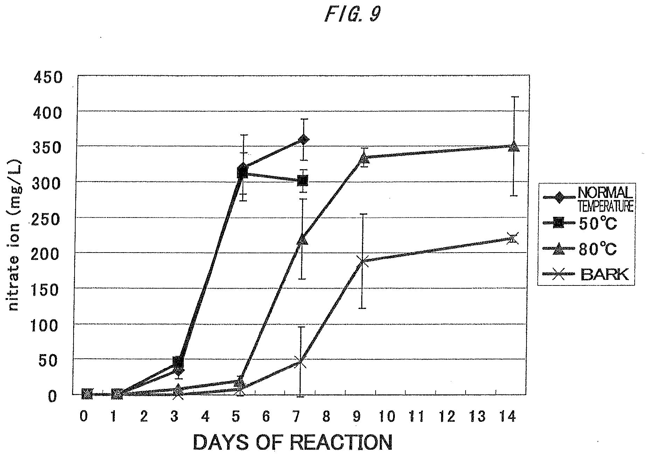

It should be noted that, as a control experiment, an experiment to perform a reaction by adding 30 mg (1 g per L of water) of bark compost (product name Golden Bark, manufactured by Shimizu Port Lumber Industry Co-operative Association) as the microorganism source was performed at the same time. FIG. 9 illustrates the results.

The results reveal that, in the case of addition of the dried microbial cells subjected to the heat treatment at 50°C for 30 minutes as the microorganism source, the reaction time to complete mineralization of the organic material into nitrate nitrogen (time until the concentration of a nitrate ion reached the peak) was 5 days.

Therefore, the dried microbial cells subjected to the heat treatment at 50°C for 30 minutes completely maintained their function as the ' inoculum of the microorganisms optimized as a catalyst for the multiple parallel mineralization' compared with the microorganisms which were left at rest at normal temperature (about 25°C) .

On the other hand, the results reveal that, in the case of addition of the dried microbial cells subj ected to the heat treatment at 80°C for 30 minutes as the microorganism source, the reaction time to complete mineralization of the organic material into nitrate nitrogen (time until the concentration of a nitrate ion reached the peak) was 9 days, which was almost the same as that in the case of using bark compost as the microorganism source.

The above-mentioned results reveal the dried microorganism cells subjected to the heat treatment at 50°C for 30 minutes had heat resistance without impairing the function as the inoculum of the microorganisms optimized as a catalyst for the multiple parallel mineralization.

Meanwhile, it was found that, even in the case of the heat treatment at 80°C for 30 minutes, the amount of a nitrate ion (nitrate nitrogen) generated after completion of the reaction was as high as that generated in the case of the microorganisms which were left at rest at a normal temperature (about 25°C), and the commercial value as the inoculum was not reduced by temporarily exposing the microorganisms to a high temperature of 80°C.

It should be noted that, in the case of using bark compost as the microorganism source, the amount of nitrate nitrogen generated after completion of the reaction was small probably because bark compost contained a large amount of organic components compared with the dried microbial cells obtained in Example 1 (Product 1-1 of the present invention), and a nitrate ion was consumed by the microorganisms using the components, resulting in decreasing the concentration of a nitrate ion to be collected.

### Industrial Applicability

The inoculum of the present invention is valuable as a technology for solving the problem of low operation efficiency (the reaction time is about two weeks, the amount of the microorganism source inoculated is large (about 5 g per L), and the upper limit of the amount of the organic material added at one time is about 2 g) in practical hydroponics using an organic fertilizer, which has attracted attention in recent years. If the inoculum provided by the present invention is used, it is possible to reduce the reaction time by half or less, to decrease the amount of the microorganism source inoculated to 4%, and to increase the amount of the organic material added to five times, resulting in drastically improving the operation efficiency.

Currently, the hydroponics using the organic fertilizer attracts attention, and farm producers who try the hydroponics increases rapidly. It is expected that much of the hydroponics, which continues to expand to 150 ha in Japan or 4000 ha in Netherlands, is replaced with hydroponics using an organic fertilizer. The inoculum provided by the present invention contributes much as a technology for supporting production activities of the farm producers, and hence, is expected to be widely used, and the market scale is very large. The inoculum of the present invention can be applied not only to hydroponics but also to water culture for display such as room gardening or rooftop gardening, and the marketability is not limited to agricultural fields.

Moreover, the present invention can be applied to a technology for producing an inorganic fertilizer using an organic waste as a raw material. The market scale of the waste recycling industry is expected to expand to 2.5 trillion yen, and the present invention has very large industrial applicability as a technology for rapidly and efficiently recycling a large amount of organic resources into inorganic nutrients.

In addition, according to the present invention, it is possible to produce a novel inoculum by using the inoculum of the present invention. The number of days for production is half of that in the case of the conventional multiple parallel mineralization, and hence, it becomes possible to produce a large amount of the inoculum rapidly. As mentioned above, the needs of the inoculum are expected to be large because the marketability of the inoculum itself is wide, and the marketability of provision of a technology for producing a large amount of the inoculum rapidly is expected to be large.

### Brief Description of Drawings

[FIGS. 1] FIGS. 1(a) to 1(c) are diagrams illustrating embodiments of the method of producing the inoculum of the microorganisms optimized as a catalyst for the multiple parallel mineralization of the present invention. Moreover, FIG.1(a) is a schematic view illustrating the method of forming and collecting the biofilm in Example 1.
[FIGS. 2] FIG. 2(a) is a schematic view illustrating an embodiment of formation of the biofilm on the solid surface in the present invention. Further, FIG. 2(b) is photographic images showing the process for forming and collecting the biofilm in Example 1. Further, FIG. 2(c) is a schematic view illustrating an embodiment of the method of collecting the biofilm in the present invention.
[FIG. 3] A graph showing the results of measurement of the concentration of a nitrate ion in Example 2.
[FIG. 4] Graphs showing the results of measurement of the concentrations of a nitrate ion, a nitrite ion, and ammonium in Example 3.
[FIG. 5] A graph showing the results of measurement of the concentration of a nitrate ion in Example 4.
[FIG. 6] A graph showing the results of measurement of the concentrations of a nitrate ion and ammonium in Comparative Example 1.
[FIG. 7] A graph showing the results of measurement of the concentration of a nitrate ion in Example 5.
[FIG. 8] A graph showing the results of measurement of the concentration of a nitrate ion in Example 6.
[FIG. 9] A graph showing the results of measurement of the concentration of a nitrate ion in Example 7.

## Claims

1. A method of producing an inoculum having the following characteristic (F);
(F) a characteristic such that the inoculum maintains its function as the inoculum of the microorganisms optimized as a catalyst for the multiple parallel mineralization when the inoculum is heated at 50 to 80°C,
wherein the multiple parallel mineralization is a reaction for successively performing degradation of an organic material into ammonium nitrogen and nitrification of ammonium nitrogen into nitrate nitrogen in the same reaction system as well as a reaction for generating nitrate nitrogen by degrading organic nitrogen contained in the organic material into ammonium nitrogen and then performing nitrification of ammonium nitrogen through nitrification in degradation of the organic material;
the method comprising:
placing water in a container that can store water therein, adding the following microorganism source (B) containing microorganisms capable of conducting a multiple parallel mineralization;
(B) at least one microorganism source selected from the group consisting of soil, compost, activated sludge, and water collected from nature;
wherein the microorganisms are selected from microorganisms capable of conducting ammonification and microorganisms capable of conducting nitrification;
maintaining an environment in water to satisfy all the following conditions (C1) to (C4), thereby culturing the microorganisms capable of conducting a multiple parallel mineralization;
(C1) a water temperature condition of 15 to 37°C:
(C2) a condition of maintaining an aerobic condition by aeration, shaking, or aeration and shaking:
(C3) a condition of adding the following organic material (D) in an amount of 0.01 to 2 g in terms of dry weight per L of the water per 1 to 14 days;
(D) at least one organic material selected from the group consisting of fish-based soluble fertilizer, corn steep liquid, oil cake, fish flour, milk, soybean cake, yeast cake, sake cake, shochu cake, and raw garbage; and
(C4) a condition of stopping addition of the organic material when a concentration of a nitrate ion generated in the water reaches 10 to 50 mg/L;
forming a biofilm on the following solid surface (E) that contacts with the water and then collecting the biofilm;
(E) at least one solid surface selected from the group consisting of a wall surface of the container and a bottom surface of the container;
and utilizing the collected biofilm as an inoculum of the microorganisms optimized as a catalyst for the multiple parallel mineralization.

2. The method of producing an inoculum according to claim 1, wherein one or more of the following materials (A1) to (A3) is immersed in the container;
(A1) a solid support comprising bamboo charcoal, charcoal, pearlite, sea sand, vermiculite, ceramic, zeolite, glass, rockwool, urethane, nylon, or a melamine resin;
(A2) a plate-like object comprising glass, acryl, plastic, ceramics, or pottery; and
(A3) a columnar structure comprising glass, acryl, plastic, ceramics, or pottery, and the biofilm is formed on the following solid surface (E-1);
(E-1) at least one solid surface selected from the group consisting of a wall surface of the container, a bottom surface of the container, a surface of the solid support, a surface of the plate-like object, and a surface of the columnar structure.

3. A method of producing an inoculum having the following characteristic (F);
(F) a characteristic such that the inoculum maintains its function as the inoculum of the microorganisms optimized as a catalyst for the multiple parallel mineralization when the inoculum is heated at 50 to 80°C, the method comprising:
placing water in a container that can store water therein, adding an inoculum obtained by the method of producing an inoculum according to claim 1 as microorganism source;
maintaining an environment in water to satisfy all the following conditions (C1), (C2), (C3-2), and (C4), thereby culturing the microorganisms capable of conducting a multiple parallel mineralization;
(C1) a water temperature condition of 15 to 37°C;
(C2) a condition of maintaining an aerobic condition by aeration, shaking, or aeration and shaking:
(C3-2) a condition of adding the following organic material (D) in an amount of 0.01 to 10 g in terms of dry weight per L of the water per 1 to 14 days;
(D) at least one organic material selected from the group consisting of fish-based soluble fertilizer, corn steep liquid, oil cake, fish flour, milk, soybean cake, yeast cake, sake cake, shochu cake, and raw garbage; and
(C4) a condition of stopping addition of the organic material when a concentration of a nitrate ion generated in the water reaches 10 to 50 mg/L;
forming a biofilm on the following solid surface (E) that contacts with the water and then collecting the biofilm;
(E) at least one solid surface selected from the group consisting of a wall surface of the container and a bottom surface of the container;
and utilizing the collected biofilm as an inoculum of the microorganisms optimized as a catalyst for the multiple parallel mineralization.

4. The method of producing an inoculum according to claim 3, wherein one or more of the following materials (A1) to (A3) is immersed in the container;
(A1) a solid support comprising bamboo charcoal, charcoal, pearlite, sea sand, vermiculite, ceramic, zeolite, glass, rockwool, urethane, nylon, or a melamine resin;
(A2) a plate-like object comprising glass, acryl, plastic, ceramics, or pottery; and
(A3) a columnar structure comprising glass, acryl, plastic, ceramics, or pottery, and the biofilm is formed on the following solid surface (E-1);
(E-1) at least one solid surface selected from the group consisting of a wall surface of the container, a bottom surface of the container, a surface of the solid support, a surface of the plate-like object, and a surface of the columnar structure.

5. The method of producing an inoculum according to claim 1, wherein the biofilm is collected by discarding a supernatant of a culture solution obtained after culturing the microorganisms capable of conducting a multiple parallel mineralization and then collecting the biofilm formed on the solid surface.

6. The method of producing an inoculum according to claim 1, comprising performing a drying treatment after collecting the biofilm.

7. The method of producing an inoculum according to claim 2, wherein the biofilm is collected by discarding a supernatant of a culture solution obtained after culturing the microorganisms capable of conducting a multiple parallel mineralization and then collecting the biofilm formed on the solid surface.

8. The method of producing an inoculum according to claim 4, comprising performing a drying treatment after collecting the biofilm.

9. An inoculum which is obtained by the method of producing an inoculum according to any one of claims 1, 2, 3, 4, 5, 6, 7 or 8, having the following characteristics (F) and (G);
(F) a characteristic such that the inoculum maintains its function as the inoculum of the microorganisms optimized as a catalyst for the multiple parallel mineralization when the inoculum is heated at 50 to 80°C; and
(G) a characteristic such that a microorganism composition is optimized as a catalyst for the multiple parallel mineralization.

10. A method of producing a fertilizer containing nitrate nitrogen as inorganic nutrients, the method comprising:
placing water in a container that can store water therein and adding the inoculum according to claim 9;
allowing a:multiple parallel mineralization to proceed in the water by maintaining an environment in the water to satisfy all the following conditions (C1), (C2), and (C3-3);
(C1) a water temperature condition of 15 to 37°C;
(C2) a condition of maintaining an aerobic condition by aeration, shaking, or aeration and shaking; and
(C3-3) a condition of adding an organic material in an amount of 20 g or less in terms of dry weight per L of the water;
providing a reaction solution containing a nitrate ion at a concentration of 100 mg/L or more;
and utilizing the resultant reaction solution as a fertilizer containing nitrate nitrogen as inorganic nutrients.

11. The method of producing a fertilizer according to claim 10, wherein the inoculum is added in an amount of 0.01 g or more per L of the water.

12. A method of cultivating a plant, comprising adding a fertilizer containing an organic material directly to the reaction solution to perform hydroponics in the reaction solution obtained according to claim 10.

## Patentansprüche

1. Verfahren zum Erzeugen eines Inokulums, das die folgenden Merkmale (F) aufweist:
(F) ein Merkmal derart, dass das Inokulum seine Funktion als das Inokulum der Mikroorganismen, die als ein Katalysator für die mehrfache parallele Mineralisierung optimiert werden, beibehält, wenn das Inokulum auf 50 bis 80 °C erhitzt wird,
wobei die mehrfache parallele Mineralisierung eine Reaktion ist, um sukzessive Abbau eines organischen Materials in Ammoniumstickstoff und Nitrifikation des Ammoniumstickstoffs in Nitratstickstoff in demselben Reaktionssystem sowie eine Reaktion zum Erzeugen von Nitratstickstoff durch Abbauen organischen Stickstoffs, der in dem organischen Material enthalten ist, in Ammoniumstickstoff und dann Ausführen von Nitrifikation des Ammoniumstickstoffs durch Nitrifikation bei Abbau des organischen Materials auszuführen;
wobei das Verfahren Folgendes umfasst:
Platzieren von Wasser in einen Behälter, der Wasser lagern kann, Hinzufügen der folgenden Mikroorganismusquelle (B), die Mikroorganismen enthält, die eine mehrfache parallele Mineralisierung ausführen können;
(B) mindestens eine Mikroorganismusquelle, die aus einer Gruppe ausgewählt ist, die aus Erdreich, Kompost, Belebtschlamm und Wasser, das aus der Natur bezogen wird, besteht;
wobei die Mikroorganismen aus Mikroorganismen ausgewählt werden, die fähig sind, Ammonifikation auszuführen, und aus Mikroorganismen, die fähig sind, Nitrifikation auszuführen;
Aufrechterhalten einer Umgebung in Wasser, um alle folgenden Bedingungen (C1) bis (C4) zu erfüllen, wodurch die Mikroorganismen, die fähig sind, eine mehrfache parallele Mineralisierung auszuführen, gezüchtet werden;
(C1) eine Wassertemperaturbedingung von 15 bis 37 °C:
(C2) eine Bedingung des Aufrechterhaltens eines aeroben Zustands durch Belüftung, Schütteln oder Belüftung und Schütteln:
(C3) eine Bedingung des Hinzufügens des folgenden organischen Materials (D) in einer Menge von 0,01 bis 2 g Trockengewicht pro Liter des Wassers per 1 bis 14 Tage;
(D) mindestens ein organisches Material, das aus der Gruppe ausgewählt wird, die aus auf Fisch basierendem löslichem Dünger, Maisquellflüssigkeit, Ölkuchen, Fischmehl, Milch, Sojabohnenkuchen, Hefekuchen, Reisweinkuchen, Shochukuchen und Rohabfall besteht; und
(C4) eine Bedingung des Stoppens des Hinzufügens des organischen Materials, wenn eine Konzentration eines Nitrat-Ions, das in dem Wasser erzeugt wird, 10 bis 50 mg/l erreicht;
Bilden eines Biofilms auf der folgenden festen Oberfläche (E), die das Wasser kontaktiert, und dann Sammeln des Biofilms;
(E) mindestens eine feste Oberfläche, die aus der Gruppe ausgewählt ist, die aus einer Wandoberfläche des Behälters und einer Bodenoberfläche des Behälters besteht;
und Einsetzen des gesammelten Biofilms als ein Inokulum der Mikroorganismen, die als ein Katalysator für die mehrfache parallele Mineralisierung optimiert sind.

2. Verfahren zum Erzeugen eines Inokulums nach Anspruch 1, wobei eines oder mehrere der folgenden Materialien (A1) bis (A3) in dem Behälter eingetaucht wird (werden);
(A1) ein fester Träger, der Bambuskohle, Holzkohle, Perlit, Seesand, Vermiculit, Keramik, Zeolith, Glass, Steinwolle, Urethan, Nylon oder Melaminharz umfasst;
(A2) ein plattenähnliches Objekt, das Glas, Acryl, Kunststoff, Keramiken oder Töpferware umfasst; und
(A3) eine säulenähnliche Struktur, die Glas, Acryl, Kunststoff, Keramiken oder Töpferware umfasst, und der Biofilm auf der folgenden festen Oberfläche (E-1) gebildet wird;
(E-1) mindestens eine feste Oberfläche, die aus der Gruppe ausgewählt ist, die aus einer Wandoberfläche des Behälters, einer Bodenoberfläche des Behälters, einer Oberfläche des festen Trägers, einer Oberfläche des plattenähnlichen Objekts und einer Oberfläche der säulenähnlichen Struktur besteht.

3. Verfahren zum Erzeugen eines Inokulums, das die folgenden Merkmale (F) aufweist:
(F) ein Merkmal derart, dass das Inokulum seine Funktion als das Inokulum der Mikroorganismen, die als ein Katalysator für mehrfache parallele Mineralisierung optimiert werden, beibehält, wenn das Inokulum auf 50 bis 80 °C erhitzt wird, wobei das Verfahren Folgendes umfasst:
Platzieren von Wasser in einen Behälter, der Wasser lagern kann, Hinzufügen eines Inokulums, das durch das Verfahren zum Erzeugen eines Inokulums nach Anspruch 1 erhalten wird, als Mikroorganismusquelle;
Aufrechterhalten einer Umgebung in Wasser, um die folgenden Bedingungen (C1), (C2), (C3-2) und (C4) zu erfüllen, wodurch die Mikroorganismen, die fähig sind, eine mehrfache parallele Mineralisierung auszuführen, gezüchtet werden;
(C1) eine Wassertemperaturbedingung von 15 bis 37 °C;
(C2) eine Bedingung des Aufrechterhaltens eines aeroben Zustands durch Belüftung, Schütteln oder Belüftung und Schütteln:
(C3-2) eine Bedingung des Hinzufügens des folgenden organischen Materials (D) in einer Menge von 0,01 bis 10 g Trockengewicht pro Liter des Wassers per 1 bis 14 Tage;
(D) mindestens ein organisches Material, das aus der Gruppe ausgewählt ist, die aus auf Fisch basierendem löslichem Dünger, Maisquellflüssigkeit, Ölkuchen, Fischmehl, Milch, Sojabohnenkuchen, Hefekuchen, Reisweinkuchen, Shochukuchen und Rohabfall besteht; und
(C4) eine Bedingung des Stoppens des Hinzufügens des organischen Materials, wenn eine Konzentration eines Nitrat-Ions, das in dem Wasser erzeugt wird, 10 bis 50 mg/l erreicht;
Bilden eines Biofilms auf der folgenden festen Oberfläche (E), die das Wasser kontaktiert, und dann Sammeln des Biofilms;
(E) mindestens eine feste Oberfläche, die aus der Gruppe ausgewählt ist, die aus einer Wandoberfläche des Behälters und einer Bodenoberfläche des Behälters besteht;
und Einsetzen des gesammelten Biofilms als ein Inokulum der Mikroorganismen, die als Katalysator für die mehrfache parallele Mineralisierung optimiert sind.

4. Verfahren zum Erzeugen eines Inokulums nach Anspruch 3, wobei eines oder mehrere der folgenden Materialien (A1) bis (A3) in dem Behälter eingetaucht wird (werden);
(A1) ein fester Träger, der Bambuskohle, Holzkohle, Perlit, Seesand, Vermiculit, Keramik, Zeolith, Glass, Steinwolle, Urethan, Nylon oder Melaminharz umfasst;
(A2) ein plattenähnliches Objekt, das Glas, Acryl, Kunststoff, Keramiken oder Töpferware umfasst; und
(A3) ein säulenähnliches Objekt, das Glas, Acryl, Kunststoff, Keramiken oder Töpferware umfasst, und der Biofilm auf der folgenden festen Oberfläche (E-1) gebildet wird;
(E-1) mindestens eine feste Oberfläche, die aus der Gruppe ausgewählt ist, die aus einer Wandoberfläche des Behälters, einer Bodenoberfläche des Behälters, einer Oberfläche des festen Trägers, einer Oberfläche des plattenähnlichen Objekts und einer Oberfläche der säulenähnlichen Struktur besteht.

5. Verfahren zum Erzeugen eines Inokulums nach Anspruch 1, wobei der Biofilm durch Verwerfen eines Überstands einer Kulturlösung, die erhalten wird, nachdem die Mikroorganismen gezüchtet wurden, die fähig sind, eine mehrfache parallele Mineralisierung auszuführen, und dann Sammeln des Biofilms, der sich auf der festen Oberfläche gebildet hat, gesammelt wird.

6. Verfahren zum Erzeugen eines Inokulums nach Anspruch 1, das das Ausführen einer Trocknungsbehandlung nach dem Sammeln des Biofilms umfasst.

7. Verfahren zum Erzeugen eines Inokulums nach Anspruch 2, wobei der Biofilm durch Verwerfen eines Überstands einer Kulturlösung, die erhalten wird, nachdem die Mikroorganismen gezüchtet wurden, die fähig sind, eine mehrfache parallele Mineralisierung auszuführen, und dann Sammeln des Biofilms, der sich auf der festen Oberfläche gebildet hat, gesammelt wird.

8. Verfahren zum Erzeugen eines Inokulums nach Anspruch 4, das das Ausführen einer Trocknungsbehandlung nach dem Sammeln des Biofilms umfasst.

9. Inokulum, das durch das Verfahren zum Erzeugen eines Inokulums nach einem der Ansprüche 1, 2, 3, 4, 5, 6, 7 oder 8 erhalten wird, das die folgenden Merkmale (F) und (G) aufweist;
(F) ein Merkmal derart, dass das Inokulum seine Funktion als das Inokulum der Mikroorganismen, die als ein Katalysator für die mehrfache parallele Mineralisierung optimiert werden, beibehält, wenn das Inokulum auf 50 bis 80 °C erhitzt wird, und
(G) ein Merkmal derart, dass eine Mikroorganismenzusammensetzung als ein Katalysator für die mehrfache parallele Mineralisierung optimiert wird.

10. Verfahren zum Erzeugen eines Düngers, der Nitratstickstoff als anorganische Nährstoffe enthält, wobei das Verfahren Folgendes umfasst:
Platzieren von Wasser in einem Behälter, der Wasser lagern kann, und Hinzufügen des Inokulums nach Anspruch 9;
Erlauben des Ausführens einer mehrfachen parallelen Mineralisierung in dem Wasser durch Aufrechterhalten einer Umgebung, in der das Wasser alle folgenden Bedingungen (C1), (C2) und (C3-3) erfüllt;
(C1) eine Wassertemperaturbedingung von 15 bis 37 °C;
(C2) eine Bedingung des Aufrechterhaltens eines aeroben Zustands durch Belüftung, Schütteln oder Belüftung und Schütteln; und
(C3-3) eine Bedingung des Hinzufügens eines organischen Materials in einer Menge von 20 g oder weniger Trockengewicht pro Liter des Wassers;
Bereitstellen einer Reaktionslösung, die ein Nitrat-Ion in einer Konzentration von 100 mg/l oder mehr enthält;
und Einsetzen der resultierenden Reaktionslösung als einen Dünger, der Nitratstickstoff als anorganische Nährstoffe enthält.

11. Verfahren zum Erzeugen eines Düngers nach Anspruch 10, wobei eine Menge von 0,01 g oder mehr pro Liter des Wassers an Inokulum hinzugefügt wird.

12. Verfahren zum Züchten einer Pflanze, das das Hinzufügen eines Düngers, der ein organisches Material enthält, direkt zu der Reaktionslösung umfasst, um Hydrokultur in der Reaktionslösung, die gemäß Anspruch 10 erhalten wird, auszuführen.

## Revendications

1. Procédé de production d'un inoculum ayant la caractéristique suivante (F) ;
(F) une caractéristique telle que l'inoculum maintienne sa fonction comme l'inoculum des microorganismes optimisé comme un catalyseur pour la minéralisation parallèle multiple lorsque l'inoculum est chauffé à 50 à 80 °C,
dans lequel la minéralisation parallèle multiple est une réaction pour effectuer successivement la dégradation d'un matériau organique en azote d'ammonium et la nitrification de l'azote d'ammonium en azote de nitrate dans le même système réactionnel ainsi qu'une réaction pour générer de l'azote de nitrate en dégradant l'azote organique contenu dans le matériau organique en azote d'ammonium et en réalisant ensuite la nitrification d'azote d'ammonium par nitrification en dégradation du matériau organique ;
le procédé comprenant :
la mise en place d'eau dans un récipient qui peut y stocker de l'eau, l'addition de la source suivante de microorganismes (B) contenant des microorganismes capables d'effectuer une minéralisation parallèle multiple ;
(B) au moins une source de microorganismes sélectionnée dans le groupe constitué de terre, de compost, de boue activée et d'eau recueillie d'une source naturelle ;
dans lequel les microorganismes sont sélectionnés parmi des microorganismes capables d'effectuer une ammonification et des microorganismes capables d'effectuer une nitrification ;
le maintien d'un environnement dans de l'eau pour répondre à toutes les conditions suivantes (C1) à (C4), en sorte de cultiver les microorganismes capables d'effectuer une minéralisation parallèle multiple ;
(C1) une condition de température de l'eau de 15 à 37 °C ;
(C2) une condition de maintien d'une condition aérobie par aération, secouage ou aération et secouage ;
(C3) une condition d'addition du matériau organique suivant (D) en quantité de 0,01 à 2 g en termes de poids sec par L de l'eau pendant 1 à 14 jours ;
(D) au moins un matériau organique sélectionné dans le groupe constitué d'un engrais soluble à base de poisson, d'un liquide de trempage, d'un tourteau, de farine de poisson, de lait, de gâteau de soja, de gâteau de levure, de gâteau de saké, de gâteau de shochu et d'ordures brutes ; et
(C4) une condition d'arrêt d'addition du matériau organique lorsqu'une concentration d'un ion de nitrate généré dans l'eau atteint 10 à 50 mg/L;
la formation d'un film biologique sur la surface solide suivante (E) qui vient en contact avec l'eau et le recueil ensuite du film biologique ;
(E) au moins une surface solide sélectionnée dans le groupe constitué d'une surface de paroi du récipient et d'une surface de fond du récipient ; et
l'utilisation du film biologique recueilli comme inoculum des microorganismes optimisé comme un catalyseur pour la minéralisation parallèle multiple.

2. Procédé de production d'un inoculum selon la revendication 1, dans lequel un ou plusieurs des matériaux suivants (A1) à (A3) est ou sont immergés dans le récipient ;
(A1) un support solide comprenant du charbon de bambou, du charbon de bois, de la perlite, du sable marin, de la vermiculite, de la céramique, de la zéolite, du verre, de la laine de roche, de l'uréthane, du Nylon ou une résine mélamine ;
(A2) un objet en forme de plaque comprenant du verre, une substance acrylique, une matière plastique, une céramique ou une poterie ; et
(A3) une structure colonnaire comprenant du verre, une substance acrylique, une matière plastique, une céramique ou une poterie et le film biologique est formé sur la surface solide suivante (E-1) ;
(E-1) au moins une surface solide sélectionnée dans le groupe constitué d'une surface de paroi du récipient, d'une surface de fond du récipient, d'une surface du support solide, d'une surface de l'objet en forme de plaque et d'une surface de la structure colonnaire.

3. Procédé de production d'un inoculum ayant la caractéristique suivante (F) ;
(F) une caractéristique telle que l'inoculum maintienne sa fonction comme inoculum des microorganismes optimisée comme catalyseur pour la minéralisation parallèle multiple lorsque l'inoculum est chauffé à 50 à 80 °C, le procédé comprenant :
la mise en place d'eau dans un récipient qui peut y stocker de l'eau en ajoutant un inoculum obtenu par le procédé de production d'in inoculum selon la revendication 1 comme source de microorganismes ;
le maintien d'un environnement dans de l'eau pour répondre à toutes les conditions suivantes (C1), (C2), (C3-2) et (C4), en sorte de cultiver les microorganismes capables d'effectuer une minéralisation parallèle multiple ;
(C1) une condition de température de l'eau de 15 à 37 °C ;
(C2) une condition de maintien d'une condition aérobie par aération, secouage ou aération et secouage ;
(C3-2) une condition d'addition du matériau organique suivant (D) en quantité de 0,01 à 10 g en termes de poids sec par L de l'eau pendant 1 à 14 jours ;
(D) au moins un matériau organique sélectionné dans le groupe constitué d'un engrais soluble à base de poisson, d'un liquide de trempage, d'un tourteau, de farine de poisson, de lait, de gâteau de soja, de gâteau de levure, de gâteau de saké, de gâteau de shochu et d'ordures brutes ; et
(C4) une condition d'arrêt d'addition du matériau organique lorsqu'une concentration d'un ion de nitrate généré dans l'eau atteint 10 à 50 mg/L;
la formation d'un film biologique sur la surface solide suivante (E) qui vient en contact avec l'eau et le recueil ensuite du film biologique ;
(E) au moins une surface solide sélectionnée dans le groupe constitué d'une surface de paroi du récipient et d'une surface de fond du récipient ; et
l'utilisation du film biologique recueilli comme inoculum des microorganismes optimisé comme un catalyseur pour la minéralisation parallèle multiple.

4. Procédé de production d'un inoculum selon la revendication 3, dans lequel un ou plusieurs des matériaux suivants (A1) à (A3) est ou sont immergés dans le récipient ;
(A1) un support solide comprenant du charbon de bambou, du charbon de bois, de la perlite, du sable marin, de la vermiculite, de la céramique, de la zéolite, du verre, de la laine de roche, de l'uréthane, du Nylon ou une résine mélamine ;
(A2) un objet en forme de plaque comprenant du verre, une substance acrylique, une matière plastique, une céramique ou une poterie ; et
(A3) une structure colonnaire comprenant du verre, une substance acrylique, une matière plastique, une céramique ou une poterie et le film biologique est formé sur la surface solide suivante (E-1) ;
(E-1) au moins une surface solide sélectionnée dans le groupe constitué d'une surface de paroi du récipient, d'une surface de fond du récipient, d'une surface du support solide, d'une surface de l'objet en forme de plaque et d'une surface de la structure colonnaire.

5. Procédé de production d'un inoculum selon la revendication 1, dans lequel le film biologique est recueilli en mettant au rebut un surnageant d'une solution de culture obtenue après culture des microorganismes capables d'effectuer une minéralisation parallèle multiple, puis le recueil du film biologique formé sur la surface solide.

6. Procédé de production d'un inoculum selon la revendication 1, comprenant la réalisation d'un traitement de séchage après recueil du film biologique.

7. Procédé de production d'un inoculum selon la revendication 2, dans lequel le film biologique est recueilli en mettant au rebut un surnageant d'une solution de culture obtenue après culture des microorganismes capables d'effectuer une minéralisation parallèle multiple, puis le recueil du film biologique formé sur la surface solide.

8. Procédé de production d'un inoculum selon la revendication 4, comprenant la réalisation d'un traitement de séchage après recueil du film biologique.

9. Inoculum qui est obtenu par le procédé de production d'un inoculum selon l'une quelconque des revendications 1, 2, 3, 4, 5, 6, 7 ou 8, ayant les caractéristiques suivantes (F) et (G) ;
(F) une caractéristique telle que l'inoculum maintienne sa fonction comme inoculum des microorganismes optimisé comme un catalyseur pour la minéralisation parallèle multiple lorsque l'inoculum est chauffé à 50 à 80 °C ; et
(G) une caractéristique telle qu'une composition de microorganismes soit optimisée comme un catalyseur pour la minéralisation parallèle multiple.

10. Procédé de production d'un engrais contenant de l'azote de nitrate comme nutriments inorganiques, le procédé comprenant :
la mise en place d'eau dans un récipient qui peut y stocker de l'eau et l'addition de l'inoculum selon la revendication 9 ;
le fait de permettre à une minéralisation parallèle multiple de s'effectuer dans l'eau en maintenant un environnement dans l'eau qui réponde à toutes les conditions suivantes (C1), (C2) et (C3-3) ;
(C1) une condition de température de l'eau de 15 à 37 °C ;
(C2) une condition de maintien d'une condition aérobie par aération, secouage ou aération et secouage ; et
(C3-3) une condition d'addition d'un matériau organique en quantité de 20 g ou moins en termes de poids sec par L de l'eau ;
la fourniture d'une solution réactionnelle contenant un ion de nitrate en concentration de 100 mg/L ou plus ; et
l'utilisation de la solution réactionnelle obtenue comme engrais contenant de l'azote de nitrate comme nutriments inorganiques.

11. Procédé de production d'un engrais selon la revendication 10, dans lequel l'inoculum est ajouté en quantité de 0,01 g ou plus par L de l'eau.

12. Procédé de culture d'une plante, comprenant l'addition d'un engrais contenant un matériau organique directement à la solution réactionnelle pour effectuer une culture hydroponique dans la solution réactionnelle obtenue selon la revendication 10.
